(19) Europäisches Patentamt
European Patent Office
Office européen des brevets

(11) EP 1 509 533 B1

# (12) EUROPEAN PATENT SPECIFICATION

(45) Date of publication and mention of the grant of the patent:
**21.10.2009 Bulletin 2009/43**

(21) Application number: **03724739.2**

(22) Date of filing: **02.06.2003**

(51) Int Cl.:
***C07F 7/04** (2006.01)*
***A61K 47/48** (2006.01)*
***G01N 30/02** (2006.01)*
***C01B 33/16** (2006.01)*
***B01D 15/08** (2006.01)*

(86) International application number:
**PCT/CA2003/000790**

(87) International publication number:
**WO 2003/102001 (11.12.2003 Gazette 2003/50)**

## (54) POLYOL-MODIFIED SILANES AS PRECURSORS FOR SILICA

MIT POLYOL MODIFIZIERTE SILANE ALS AUSGANGSSTOFFE FÜR KIESELSÄURE

SILANES MODIFIES PAR DES POLYOLS EN TANT QUE PRECURSEURS DE SILICE

(84) Designated Contracting States:
**AT BE BG CH CY CZ DE DK EE ES FI FR GB GR HU IE IT LI LU MC NL PT RO SE SI SK TR**

(30) Priority: **31.05.2002 US 384084 P**

(43) Date of publication of application:
**02.03.2005 Bulletin 2005/09**

(73) Proprietor: **McMaster University**
**Hamilton, Ontario L8P 0A1 (CA)**

(72) Inventors:
- **BROOK, Michael, A.**
  **Ancaster, Ontario L9G 4M4 (CA)**
- **BRENNAN, John, D.**
  **Dundas, Ontario L9H 3V5 (CA)**
- **CHEN, Yang**
  **Hamilton, Ontario L8S 4N2 (CA)**

(74) Representative: **Chapman, Paul Gilmour et al**
**Marks & Clerk LLP**
**Aurora**
**120 Bothwell Street**
**Glasgow**
**G2 7JS (GB)**

(56) References cited:
**WO-A-01/58562 US-A- 6 090 448**

- **GILL I ET AL: "ENCAPSULATION OF BIOLOGICALS WITHIN SILICATE, SILOXANE, AND HYBRID SOL-GEL POLYMERS: AN EFFICIENT AND GENERIC APPROACH" JOURNAL OF THE AMERICAN CHEMICAL SOCIETY, AMERICAN CHEMICAL SOCIETY, WASHINGTON, DC, US, vol. 120, 1998, pages 8587-8598, XP000861908 ISSN: 0002-7863 cited in the application**
- **BAKUL C DAVE ET AL: "SOL-GEL ENCAPSULATION METHODS FOR BIOSENSORS" ANALYTICAL CHEMISTRY, AMERICAN CHEMICAL SOCIETY. COLUMBUS, US, vol. 66, no. 22, 15 November 1994 (1994-11-15), pages 1120A-1127A, XP000483982 ISSN: 0003-2700**
- **IQBAL GILL: "Bio-Doped Nanocomposite Polymers: Sol-Gel Bioencapsulates" CHEMISTRY OF MATERIALS, vol. 13, no. 10, October 2001 (2001-10), pages 3404-3421, XP002250511 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The invention relates to silica and the preparation of silica from polyol-modified silanes under mild conditions.

### BACKGROUND OF THE INVENTION

Silica in its various forms comprises more than half of the earth's crust.[1] While many applications utilize silica in its natural forms, a wide variety of other morphological structures of silica may be prepared by other routes for other uses. Thus, high surface area silica (fumed silica), used in the reinforcement of silicone polymers, is prepared by the controlled burning of chlorosilanes in a hydrogen flame; precipitated silicas, derived from sodium silicate, are used as chromatographic supports and colloidal silica of dimensions 50-1000 nm can be prepared in almost monodisperse form by the Stöber process.[2] The latter process, which utilizes sol-gel chemistry, has been exploited in a number of situations where monodispersity is required, such as in the colloidal crystals used by Ozin for wave guides.[3]

The sol-gel process has also been recently exploited for catalyst synthesis because it provides the ability to control inner structure in silicas. Thus, surfactant contaminants such as long chain alkylammonium salts template the formation of mesostructured silicas with well-defined pore structures such as MCM-41.[4,5] The sizes of the pores may be controlled by the nature of the contaminant, a fact that has permitted the preparation of a family of catalytically active silicas. The control of morphology leads to the possibility of doping these silicas to change their catalytic properties.

It was recognized in the 1980s that the mild conditions used for preparing sol-gel silicas were compatible with the incorporation of fragile compounds, such as proteins, into the silica. A wide variety of proteins, enzymes[6] and other sensitive biopolymers including DNA and RNA, and complex systems including whole plant, animal and microbial cells have subsequently been entrapped in silica.[7] In these structures, the silica serves to protect the entrapped material, to some extent, from external environments, improving its longevity as judged by biological activity. These materials are of interest as catalysts and as biosensors.[8]

The basic building block for protein-doped silicas has traditionally been tetraethoxysilane (TEOS) or tetramethoxysilane (TMOS). The chemistry of these inexpensive and readily available materials is well understood. Scheme 1 below shows the hydrolysis/condensation steps involved in the conversion of tetraalkoxysilanes into silica.[9,10,11] It has been demonstrated that either acidic or basic conditions are required for the hydrolysis part of the two step process, whereas condensation is facilitated near neutrality (see Figure 1 which shows the pH dependencies of hydrolysis (H) and condensation (C) and dissolution (D) for a TEOS:$H_2O$ ratio of 1.5 in the formation of silica.[9,12] The morphology of the silica produced under different pH regimes is quite different as acid-catalyzed hydrolysis condensation generally leads to crosslinked arrays of long fibrils, whereas base-catalyzed processes lead to highly crosslinked three-dimensional structures that are then embedded in amorphous silica (the raison bun model).[9]

**Scheme 1**

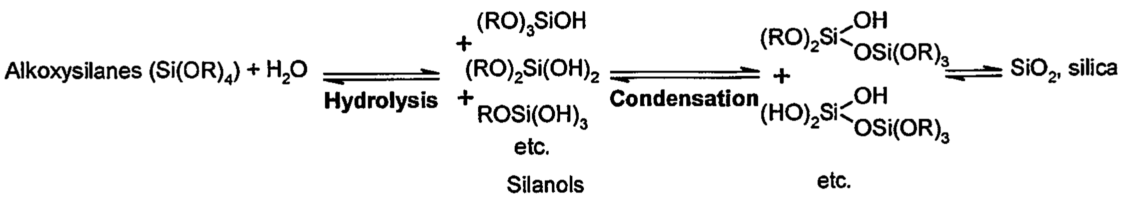


While TEOS offers many advantages as a starting material for silica, there are accompanying disadvantages when a protein-(or other biomolecule)-embedded silica is the desired product. The optimal acidic or basic conditions required to implement the sol-gel chemistry are in general incompatible with protein stabilization. Therefore, a complex sequence of pH regimes is typically utilized to prepare protein-doped silica. The sol-gel process is generally initiated at low pH in the absence of protein, and then the pH of the sol is changed to near neutrality by the addition of protein in buffer, and the gelation allowed to continue. Reproducing these pH protocols can be challenging.

TEOS has other features that compromise its use for the preparation of protein-doped silicas. First, the protein denaturant, ethanol, is formed as a byproduct of the reaction. The protein stability thus hinges on the ability to remove the ethanol from the silica matrix. Second, the cure characteristics of the silica formed from TEOS are incompatible with

long-term stability of the protein. The optimal crosslinking density that is compatible with a stabilized and immobilized protein occurs long before the cure process has completed. Over time, TEOS-derived gels shrink extensively frequently leading to cracking of the brittle matrix and concomitant protein denaturation.

The combination of silicon with polyols was first reported in the 1950s.[13] At that time, it was noted that the hydrolytic stability of such species was too low for the compounds to be of general use.[13,17,18,19] It is now known that for the preparation of silica, at least, hydrolytic instability of the starting materials is desired. A further advantage of the use of silicon polyol precursors is the fact that upon hydrolysis, the resulting polyol, unlike ethanol, should not be deleterious to protein structure, and in some cases may even stabilize proteins.[20] The innocuous nature of polyols in biological systems is further suggested by the recent report that sugar acid:silane complexes may act as the transportable form of silica precursors in the biogenesis of silica in organisms such as diatoms.[21]

WO 99/24517 discloses a coating composition comprising components I-VI, wherein component I is the reaction product of at least one organic polyol and a silicon-containing compound of the formula $R_x$-Si-$OR_{4-x}$ where R is alkyl or aryl.

To exploit the innocuous nature of polyols, researchers recently prepared poly(glyceryl silicate) (PGS) as the silica matrix for bioencapsulation of protein.[20] The preparation of PGS began with the partial hydrolysis and condensation of tetramethylorthosilicate (TMOS) to form poly(methyl silicate) (PMS). The PMS was then transesterified with glycerol in the presence of hydrochloric acid or poly(antimony(III) ethylene glycoxide) as a catalyst to form PGS. The PGS then underwent hydrolysis and gellation to form silica hydrogels which were then aged, washed with water to remove the glycerol and dried to form mesoporous silica xerogels. Although, the PGS-derived silica xerogels exhibited both reduced shrinkage and reduced pore collapse,[20] the need to use hydrochloric acid or poly(antimony(III) ethylene glycoxide) as a catalyst in the preparation of PGS is problematic as such contaminants may not be compatible with protein stabilization. It should be noted that no experimental protocol or structural characterization of glycerol:silane compounds ($Si(Gly)_{2-4}$)) was provided in this report.[20]

Thus, there remains a need to develop yet more gentle methods for the preparation of silicas from well-defined alkoxysilane precursors that provide: stabilizing environments for the protein; the absence of possibly deleterious catalysts, silica monoliths with low shrinkage characteristics; the possibility of controlling rates of cure by means other than pH; and the possibility of controlling the morphology, including porosity and pore structure, of the protein-containing silica.

## SUMMARY OF THE INVENTION

The present inventors have developed a method of preparing organic polyol-modified silane precursors useful for the preparation of biopolymer-compatible silicas. The method does not require the use of catalysts and involves the use of organic polyols that are compatible with proteins or other biomolecules. The silane precursor compositions prepared using the method of the invention are novel as they do not contain contaminants such as Lewis or BrØnsted acid catalysts that may not compatible with proteins.

Accordingly, the present invention involves a method of preparing organic polyol silanes comprising:

(a) combining at least one alkoxysilane with one or more organic polyols under conditions sufficient for the reaction of the alkoxysilane(s) with the organic polyol(s) to produce polyol-substituted silanes and alcohols without the use of a catalyst; and
(b) optionally, removal of the alcohols.

In embodiments of the present invention, the organic polyol is biomolecule compatible and is derived from natural sources. In particular, the organic polyol is selected from sugar alcohols, sugar acids, saccharides, oligosaccharides and polysaccharides.

The present invention further relates to novel organic polyol silane compounds, which are useful as precursors to biomolecule compatible silica, prepared using the method of the invention.

The present invention further includes an organic polyol silane composition consisting of one or more alkoxysilanes, one or more organic polyols and, optionally, a solvent.

The invention further includes silica, for example silica monoliths or silica gels, prepared using an organic polyol silane precursor of the invention and methods for their preparation. Accordingly, the present invention also relates to a method for preparing silica monoliths comprising hydrolyzing and condensing a polyol silane precursor prepared according to the method of the present invention at a pH suitable for the preparation of a silica monolith, and/or compatible with proteins or other biomolecules that may be optionally included, and allowing a gel to form. In embodiments of the invention, the silica monoliths are prepared using sol-gel techniques.

In still further embodiments, the overall pore size, total porosity and surface area of the silica gels can be changed by adding a variety of different additives. Accordingly, the present invention relates to a method for preparing a silica gel comprising:

(a) hydrolyzing and condensing a polyol silane precursor prepared according to the method of the present invention at a pH suitable for the preparation of a silica gel and in the presence of one or more additives; and
(b) allowing a gel to form,

In embodiments of the invention the one or more additives are independently selected from the group consisting of multivalent ions and hydrophilic polymers

Also, included within the scope of the present invention is a use of a silica monolith comprising an active biomolecule entrapped therein to quantitatively or qualitatively detect a test substance that reacts with or whose reaction is catalyzed by said encapsulated active biomolecule, and wherein said silica monolith is prepared using a method of the invention. Further the present invention relates to a method for the quantitative or qualitative detection of a test substance that reacts with or whose reaction is catalyzed by an active biomolecule, wherein said active biomolecule is encapsulated within a silica monolith, and wherein said silica monolith is prepared using a method of the invention. The quantitative/qualitative method comprises (a) preparing a silica monolith comprising said active biological substance entrapped within a silica matrix prepared using a method of the invention; (b) bringing said biomolecule-comprising silica monolith into contact with a gas or aqueous solution comprising the test substance; and (c) quantitatively or qualitatively detecting, observing or measuring the change in one or more optical characteristics in the biomolecule entrapped within the silica monolith.

Also included in the present invention is a method of storing a biologically active biomolecule in a silica matrix, wherein the silica matrix is prepared using a method of the present invention.

The silica monoliths prepared using the method of the invention may also be used in chromatographic applications. For the preparation of a chromatographic column, the silica precursor and, optionally one or more additives and/or a biomolecule, may be placed into a chromatographic column before gelation occurs.

The present invention therefore relates to a method of preparing a chromatographic column comprising:

(a) placing a polyol silane precursor prepared using a method of the invention, in a column, optionally in the presence of one or more additives and/or a biomolecule; and
(b) hydrolyzing and condensing the polyol silane precursor in the column.

Other features and advantages of the present invention will become apparent from the following detailed description. It should be understood, however, that the detailed description and the specific examples while indicating preferred embodiments of the invention are given by way of illustration only, since various changes and modifications within the spirit and scope of the invention will become apparent to those skilled in the art from this detailed description.

## BRIEF DESCRIPTION OF THE DRAWINGS

The invention will now be described in relation to the drawings in which:

**Figure 1** is prior art and shows the pH dependencies of hydrolysis (H) and condensation (C) and dissolution (D) for a TEOS:$H_2O$ ratio of 1.5 in the formation of silica.[12,23].
**Figure 2** is a graph of the relationship between the gel time and initial pH when diglycerylsilane (DGS) is used as the silica precursor.
**Figure 3** is a transmission electron microscopic (TEM) image (EtOH/$H_2O$ using constant infusion of gaseous $NH_{3;}$ vertical scale bar = 100 nm) of silica that was prepared from DGS.
**Figure 4 A** is a graph showing the effect of different alcohols on gelation time of TEOS derived silica and **B** is a graph showing the effect of glycerol on gelation time of DGS-derived silica.
**Figure 5** is a graph showing the shrinkage of TEOS-derived and DGS-derived gels over time.
**Figure 6** is a graph showing the results of the thermogravimetric (TG) analyses of triethoxysilane (TEOS), DGS and monosorbitylsilane (MSS) derived silica gels.
**Figure 7** is a graph showing the results of the thermogravimetric (TG) analyses of DGS derived silica with and without presoaking in water.
**Figure 8A** is a graph showing absorbance as a function of S-2222 concentration related to the activity of Factor Xa in solution and **Figure 8B** is a graph showing absorbance as a function of the inverse of the S-2222 concentration related to the activity of Factor Xa in DGS-derived silica gel matrix. Open symbols are values obtained in solution, closed symbols are values obtained in DGS.
**Figure 9** is a graph showing the activity of Factor Xa over time in DGS and TEOS-derived silica.
**Figure 10** is a graph showing the pore size distribution of DGS-derived gels containing no additives, $MgCl_2$ and albumin (protein).
**Figure 11** is a graph showing the effect of PEO on the pore size of DGS-derived silica.

## DETAILED DESCRIPTION OF THE INVENTION

### (I) Definitions

The term "gel" as used herein refers to solutions (sols) that have lost flow.

The term "gel time" as used herein is the time required for flow of the sol-gel to cease after addition of the buffer solution, as judged by repeatedly tilting a test-tube containing the sol until gelation occurred.

The term "cure" as used herein refers to the crosslinking process, the continued evolution of the silica matrix upon aging of the silica following gelation, until the time when the gel is treated (e.g., by washing, freeze drying etc.).

The term "PEO" as used herein means polyethylene oxide which has the formula $HO-(CH_2CH_2O)_n-H$, wherein n can vary from one to several hundred thousand.

### (II) Polyol-substituted Silanes

The present inventors have prepared several different organic polyol-silane precursors by transesterifying TEOS or TMOS with organic polyols. These precursors are mixtures of materials with well-defined constitutions (i.e., controlled ratios of organic residues to silicon). Polyols were used to replace ethoxy or methoxy groups on silanes to give protein-friendly starting materials. These polyols undergo transesterification with TEOS and TMOS in a variety of silane/alcohol ratios without the need for catalysts; the lower alcohols were simply removed by distillation.

Accordingly, the present invention involves a method of preparing organic polyol silanes comprising:

(a) combining at least one alkoxysilane with one or more organic polyols under conditions sufficient for the reaction of the alkoxysilane(s) with the organic polyol(s) to produce polyol-substituted silanes and alcohols without the use of a catalyst; and
(b) optionally, removal of the alcohols.

In embodiments of the invention, the method of preparing organic polyol silanes comprises:

(a) combining an alkoxysilane with an organic polyols under conditions sufficient for the reaction of the alkoxysilane with the organic polyol to produce polyol-substituted silanes and alcohols without the use of a catalyst; and
(b) optionally, removal of the alcohols.

Alkoxysilane starting materials that may be used in the method of the invention include those which have the formula: $R_4Si$, where R is any alkoxy group that can be cleaved from silicon under the conditions for performing the method of the invention. The R groups need not all be the same, therefore it is possible for one or more of the R groups to be different. In embodiments of the invention the alkoxysilane is a heterogenous or homogenous alkoxysilane derived from methanol, ethanol, propanol and/or butanol. In further embodiments of the invention, all four R groups are selected from methoxy, ethoxy, propoxy and butoxy. In still further embodiments, the alkoxysilane is selected from tetraethoxysilane (TEOS) and tetramethoxysilane (TMOS).

The organic polyols may be selected from a wide variety of such compounds. By "polyol", it is meant that the compound has more the one alcohol group. The organic portion of the polyol may have any suitable structure ranging from straight and branched chain alkyl and alkenyl groups, to cyclic and aromatic groups. For the preparation of biomolecule compatible silicas, it is preferred for the organic polyol to be biomolecule compatible. By "biomolecule compatible" it is meant that the polyol either stabilizes proteins and/or other biomolecules against denaturation or does not facilitate denaturation. The term "biomolecule" as used herein means any of a wide variety of proteins, peptides, enzymes and other sensitive biopolymers including DNA and RNA, and complex systems including whole plant, animal and microbial cells that may be entrapped in silica. In embodiments of the invention, the biomolecule is a protein, or fragment thereof.

It is preferred for the polyol to be derived from natural sources. Particular examples of preferred polyols include, but are not limited to sugar alcohols, sugar acids, saccharides, oligosaccharides and polysaccharides. Simple saccharides are also known as carbohydrates or sugars. Carbohydrates may be defined as polyhydroxy aldehydes or ketones or substances that hydroylze to yield such compounds. The polyol may be a monosaccharide, the simplest of the sugars or carbohydrate. The monosaccharide may be any aldo- or keto-triose, pentose, hexose or heptose, in either the open-chained or cyclic form. Examples of monosaccharides that may be used in the present invention include, but are not limited to, allose, altrose, glucose, mannose, gulose, idose, galactose, talose, ribose, arabinose, xylose, lyxose, threose, erythrose, glyceraldehydes, sorbose, fructose, dextrose, levulose and sorbitol. The polyol may also be a disaccahride, for example, but not limited to, sucrose, maltose, cellobiose and lactose. Polyols also include polysaccharides, for example, but not limited to dextran, (500-50,000 MW), amylose and pectin. Other organic polyols that may be used include, but are not limited to glycerol, propylene glycol and trimethylene glycol.

Specific examples of organic polyols that may be used in the method of the invention, include but are not limited to, glycerol, sorbitol, maltose, trehelose, glucose, sucrose, amylose, pectin, lactose, fructose, dextrose and dextran and the like. In embodiments of the present invention, the organic polyol is selected from glycerol, sorbitol, maltose and dextran. Some representative examples of the resulting polyol modified silanes prepared using the method of the invention include diglycerylsilane (DGS), monosorbitylsilane (MSS), monomaltosylsilane (MMS), dimaltosylsilane (DMS) and a dextran-based silane (DS). One of skill in the art can readily appreciate that other molecules including simple saccharides, oligosaccharides, and related hydroxylated compounds can also lead to viable silica precursors. Higher molecular weight water soluble polyol polymers do not leach from the silica, once formed, and therefore are a specific embodiment of the invention.

In embodiments of the invention, the conditions sufficient for the reaction of the alkoxysilane with the organic polyol to produce polyol-substituted silanes and alkoxy-derived alcohols without the use of a catalyst include combining (in any order) the alkoxysilane(s) and organic polyol(s), either neat or in the presence of a polar solvent (for example DMSO) and heating to temperatures in the range of about 90 °C to about 150 °C, suitably about 100 °C to about 140 °C, more suitably about 110 °C to about 130 °C, for about 3 hours to about 72 hours, suitably about 10 hours to about 48 hours. A person skilled in the art would appreciate that reaction times and temperatures may vary depending on the identity and amounts of specific starting materials used and could monitor the reaction progress by known means, for example NMR spectroscopy, and adjust the conditions accordingly. It has been found that when lower polyols (typically less that 3-5 carbon atoms) were used in the method of the invention, solvents were not required. Higher molecular weight polyols (>6 carbon atoms) typically required the presence of polar solvents such as DMSO in order to afford partly or completely homogeneous reaction conditions. When reacted with sugars, the TEOS-derived polyol DMSO solutions were initially heterogeneous, but became homogeneous after heating at 110-120 °C for about one hour. The alkoxy alcohol formed as a by-product and/or any solvent used in the method of the invention may be removed by any convenient means, for example, by distillation. The polyol silane product may optionally be isolated by known techniques, for example by evaporation of solvent and/or recrystallization. In embodiments of the invention, the method of preparing an organic polyol silane further comprises the stop of removal of the alkoxy alcohols.

When stoichiometrically balanced (that is, when the molar equivalents of alcohol groups on the polyols equal or exceed those of the alkoxy groups on the alkoxysilane, typically 4), complete alcohol exchange was demonstrated by $^{1}H$ NMR and $^{13}C$ NMR; no residual methoxy/ethoxy/etc. groups in the product were detected (see Examples 1-4). If exceptional care was taken to dry the solvents and precursors, it was possible to elicit transesterification to give essentially only new $Q^0$ species - Q refers to various $Si(O_{4/2})$ species.[24] Otherwise, transesterification was accompanied by condensation, as observed using $^{29}Si$ NMR, to give $Q^1$, $Q^2$ and $Q^3$ species. Note that no catalyst is necessary for the transesterification of silanes, avoiding contamination by these catalysts in the resulting silica.

The method of the invention can be carried out in a variety of silane/alcohol ratios. Thus when using one type of polyol, several different polyol silanes may be formed depending on the ratio of starting alkoxysilane to polyol. The stoichiometric ratio of silicon to polyol in these products affects their rate of hydrolysis and the rate of cure to give silica. Thus, the desirable properties of these compounds include the possibility of tuning the speed with which silica forms, and the ultimate morphology of the silica. Compounds comprising several alcohol/silane ratios were prepared and their hydrolytic behavior examined and described herein (see Tables 1-4 and Examples 1-4). It is understood that other polyol silanes, and ratios of polyols to silane are readily prepared and not excluded from the scope of the present invention.

The present invention provides the first example of polyol silane compounds and compositions which lack acidic or other catalytic contaminants. Such contaminants can affect the silica cure, and also may not be compatible with biomolecules. Further, the polyol silanes of the present invention possess characteristics that allow the morphology of the resulting silica to be controlled.

Accordingly, the present invention includes a polyol silane compound prepared by

(a) combining at least one alkoxysilane with one or more organic polyols under conditions sufficient for the reaction of the alkoaysilane(s) with the organic polyol(s) to produce polyol-substituted silanes and alcohols without the use of a catalyst; and

(b) optionally, removal of the alcohols.

The present invention further includes an organic polyol silane composition consisting of one or more alkoxysilanes, one or more organic polyols and, optionally, a solvent. In preferred embodiments of the invention the organic polyol is biomolecule compatible.

In embodiments of the present invention, there is included an organic polyol silane wherein the organic polyol is biomolecule compatible. In further embodiments of the invention the organic polyol is derived from sugar alcohols, sugar acids, saccharides, oligosaccharides and polysaccharide. In further embodiments of the invention the organic polyol silane is free of acidic and other catalytic contaminants. By "free of acidic and other catalytic contaminants" it is meant that the silane contains less than 5%, preferably less than 2%, most preferably less than 1%, of acids and other

catalytic components. By "acids and other catalytic components" it is meant any such species that is used to catalyze the hydrolysis and condensation of alkoxysilanes and alcohols. Specific examples of such species include BrØnsted acids, such as hydrochloric acid, Lewis acids and other catalysts such as poly(antimony(III) ethylene glycoxide.

In specific embodiments of the present invention, there is included an organic polyol silane selected from the group consisting of monoglycerylsilane, diglycerylsilane, tetraglycerylsilane, sorbitylsilane(2:3), monosorbitylsilane, disorbitylsilane, maltosyldisilane, monomaltosylsilane, dimaltosylsilane, quadridextransilane, demidextransilane and dextransilane (as found in Tables 1-4).

**(III) Silicas Prepared from Polyol-substituted Silanes**

The present invention further relates to the preparation of monolithic mesoporous silica under mild conditions from the organic polyol silanes and organic polyol silane compositions of the invention. Unlike the commonly used silica starting material, TEOS ($Si(OEt)_4$), the sol-gel hydrolysis and cure of the organic polyol derivatives of the present invention are not very sensitive to pH as similar rates of gelation were observed over a pH range of about 5.5-11. In addition, the rate of hydrolysis and condensation is modified by several factors including: the specific polyol, the polyol:silane ratio, the pH, ionic strength and the presence of additional polyols. For example, the gelation rate could be retarded by the use of starting materials derived from higher molecular weight polyols or by the addition of organic polyols to the curing mixture. The shrinkage of the silica monoliths prepared from the polyol modified silane precursors of the invention was lower in comparison to TEOS-derived gels, possibly because of the residual incorporation of the sugar alcohols. The shrinkage also depends strongly on the specific polyol incorporated in the precursor silane, with higher polyols (i.e. polyols having > 6 carbon atoms) leading to reduced shrinkage. These alcohols could be removed by extraction with water, but even after the removal of the sugars, the gels did not shrink if they were allowed to remain swollen with water. Thus, greater control over reaction rate, shrinkage and resulting silica morphology is available with the organic polyol silanes of the present invention than when silica is prepared from TEOS. Further, the polyol silane silica precursors of the present invention do not contain acidic or other catalytic contaminants that can affect the silica cure.

The properties of these polyol-derived silanes lend themselves to the preparation of silica under conditions that are compatible with biomolecules. The hydrolysis reactions release only the polyols, for example the sugars, sugar alcohol(s), sugar acids, oligo- or polysaccharides which typically stabilize, or at least are not detrimental to protein tertiary structure.[25]

The present invention therefore further includes a method for preparing silica monoliths comprising hydrolyzing and condensing a polyol silane precursor prepared according to the method of the present invention at a pH suitable for the preparation of a silica monolith, and/or compatible with proteins or other biomolecules that may be optionally included, and allowing a gel to form.

The hydrolysis and condensation of the polyol silane precursors may suitably be carried out in aqueous solution. Suitably, a homogenous solution of precursor, in water is used. Sonication may be used in order to obtain a homogeneous solution. The pH of the aqueous solution of polyol silane precursor may then be adjusted so that formation of a gel (the monolith) occurs. Suitably, the pH may be in the range of about 5.5 - 11. The pH may be adjusted by the addition of suitable buffer solutions. For the embedding of biomolecules into the gel, the buffer may further comprise the desired biomolecule.

The invention further includes silica monoliths prepared using the method of the invention. The silica monoliths prepared using the method of the invention are desirably biocompatible as they do not contain any residual catalysts (for example acids or Lewis acidic metal salts) from the preparation of the polyol silane precursors. Accordingly, the monoliths may further comprise a biomolecule.

Unlike the behavior of TEOS shown in Figure 1, polyol modified silanes show very different cure behaviors as a function of pH (see Figure 2). Shortly after dissolving the polyol:silane compounds in water (typically < 10 minutes), irrespective of the starting pH (over the range from 5.5-11), the $^1H$ NMR and $^{13}C$ NMR show only the sugar alcohol and there is no evidence of the formation of complex alcohols nor, therefore, of complex silanes. The nature of the silicon species during and immediately after hydrolysis has not been ascertained. In contrast to the behavior of TEOS, at a given ionic strength, the gel point for DGS is identical within experimental error over this pH range (see Figure 2, Example 8), with or without the addition of buffer (or protein-containing buffer). Small variations in the conditions of gelation, ionic strength and sample history (particularly hydration) can affect the rate. In all these cases, monolithic silica (optically clear, glass-like) materials resulted from the hydrolysis/condensation of these polyol silanes over this pH range (see Examples 5-7). Proteins or other biomolecules may be optionally included at any point prior to gelation (see Examples 12, 13). Particulate rather than monolithic silica is prepared at much higher pHs (for example pH> 12, see Figure 3, which shows particulate sol-gel derived silica).

Several factors affect the rate of cure of polyol modified silane precursors including the ratio of polyol to silicon in the starting materials, the ratio of water to silane used in the sol-gel chemistry, the presence of other diluents including alcohols, and the ionic strength of the water. The higher the polyol/silicon stoichiometric ratio in the starting material,

the slower is the rate of cure (e.g., the rate of cure followed the order: $Si(sorbitol)_4$ < $Si(sorbitol)_3$ < $Si(sorbitol)_2$ < Si (sorbitol)). This can be clearly seen in the cure characteristics of glycerol, sorbitol, maltose and dextran-based silanes (see Table 5). Of course, the gelation rates are also dependent on the nature of the container and the exposed surface area (where comparisons were made in the results below, they were made under identical experimental conditions).

Generally speaking, under the same pH profile, the polyol-derived silanes DGS, MSS and Ma1S2 gelled more quickly than TEOS, but at comparable rates to one another. However, polyol silanes derived from higher polyols cured more slowly than lower alcohols (i.e., the cure of Ma1S2 < DGS). The cure of the sol derived from pure DS was generally very slow; at lower ionic strengths cure did not take place. Irrespective of pH, as the silane is further and further diluted by water, the rate of cure is reduced as anticipated. By contrast, an increase in ionic strength increases the rate of gelation (Table 5).

The cure can also be retarded by the addition of extra polyols to the aqueous media. Performing the hydrolysis of DGS under otherwise identical conditions in the presence of additional mono-, di- and triols clearly showed this effect (see Figure 4B, Examples 9, 10). Similar effects were observed with TEOS (see Figure 4A). Thus, it is possible to control the rate of cure by addition of polyols, water concentration and pH.

Particularly convenient starting materials were found to be those with approximately a silicon/polyol residue ratio of 1:1: for example, 1 Si : 2 glycerol DGS; 1 Si : 1 sorbitol MSS; 2 Si : 1 mannitol Ma1S2, respectively. In the present examples, DGS, MSS and Ma1S2 were particularly convenient because of the ease of removing contaminants (ethanol or methanol) during their formation, the compatibility of the hydrolysis by-products with proteins, the ability to perform the reaction at a wide variety of pHs including neutrality, the reduced shrinkage and optical clarity of the resulting silicas (see below) and the rate of cure.

In addition to these control features, the degree of shrinkage can be modified on demand. Silica gels prepared from TEOS are known for their susceptibility to shrinkage. After drying in air over extended periods of time, %volume/volume shrinkages of up to 85% were observed. As shown by the graph in Figure 5, the shrinkage of DGS gel is smaller than that of TEOS gel during the period of aging.

For example, 100 hours after the gelation time, the shrinkage of DGS gel is 17%, the shrinkage of TEOS gel is 29%. Shrinkage is relative to the initial volume of the fresh hydrogel and was determined according to the equation:

$$\% V'/V = (\text{initial volume} - \text{present volume}) \div \text{initial volume} \times 100\%$$

In this procedure, the volume of the freshly prepared monolithic hydrogel (initial volume) was measured first, and then the volume of monolithic gel (present volume) was measured by assessing water displacement by the monolith at subsequent aging times. This was generally accompanied by embrittlement and cracking. The shrinkage of the monoliths prepared from glyceryl, sorbityl and dextran-based silanes materials was compared to the shrinkage of monoliths prepared from TEOS. If allowed to dry over 10 days under atmospheric exposure, shrinkages of DGS-derived gels of up to 65% (and MSS-derived gels of up to 50%) were noted. Thus, there is an inverse correlation between the polyol molecular weight and monolith shrinkage. Essentially no shrinkage was noted in closed containers or under water. In the absence of complete experimental details, it is difficult to compare these values to those of previously reported poly (glycerylsilicate)-derived silica xerogels[20] for which drying in air for 96 hours was reported to lead to 4-29% shrinkage, and freeze drying led to 16-40%, shrinkage.

While not wishing to be limited by theory, the reduced shrinkage observed for gels of the present invention (compared to TEOS-derived gels) may be a result of residual sugar alcohol in the silica during formation of the gel. Whereas TEOS-derived silica showed essentially no weight loss on heating, thermogravimetric analysis (TGA) of the DGS compounds showed that they lost up to 50% of their weight upon heating. Similar losses were observed with MSS (see Figure 6, Example 11) and other sugar silanes. The sugars could be readily removed from the cured silica by washing with water, though not by freeze-drying. The TGAs of the freeze-dried silica derived from DGS depended on whether the monoliths were washed with water. Without washing, residual organic molecules are lost thermally starting at about 200 °C, whereas after washing, there is essentially no weight loss on heating (see Figure 7), as there are no residual sugars to be removed by pyrolysis. Once the sugars and sugar-derived compounds were removed by washing, an increase in shrinkage was observed upon drying in air.

The monoliths formed from polyol modified silanes are particularly suitable for inclusion of proteins, which remain natured, and in the case of enzymes, completely active. The DGS derived silica monoliths of the present invention were tested for viable protein entrapment with Factor Xa, a blood clotting protein, which is exemplary of a series of enzymes. Factor Xa operates by selectively cleaving the Arg-/-Thr and then Arg-/-Ile bonds in prothrombin to form thrombin. Two types of assays are generally used for monitoring Factor Xa activity, i.e., clotting assay and chromogenic assay.[26,27] The chromogenic assay, where synthetic substrates such as S-2222 and S-2337 are used, allows one to assay the impact of Factor Xa on different steps in the coagulation process (Figure 8). Using S-2222 as the substrate,

the reaction catalyzed by Factor Xa is shown in Scheme 2.

Scheme 2

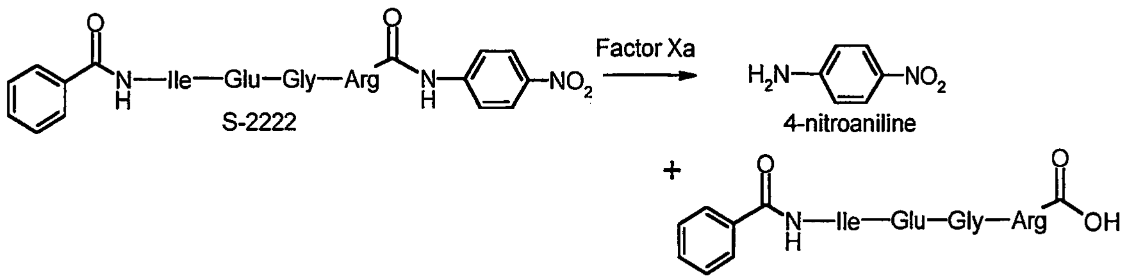


The $K_m$ value of Factor Xa in DGS is only slightly higher than in solution (see Example 12 and Table 6), indicating that the affinity of the active site for substrate is almost unaffected by encapsulation in DGS-derived silica. The enzyme turnover number ($k_{cat}$) and catalytic efficiency ($k_{cat}$/ $K_m$) shown in Table 6 appear to be unaffected by the encapsulation in the DGS-derived silica. It has been found that upon encapsulation in DGS-derived sol-gel matrix, $K_m$ values typically increase and $k_{cat}$ values decrease, which is consistent with weaker binding and slower reaction kinetics for the entrapped protein.[28,29,30,31] The reported $K_m$ value of an enzyme upon entrapment can be as high as 100 times and the $k_{cat}$ value can be as low as 4600 times in comparison to those same values obtained when the enzyme is in solution. While not wishing to be limited by theory, this may largely be due to the slow diffusion of the substrate in the sol-gel matrix and the partial inaccessible portion of the enzyme. In the case of the present invention, no significant change in both $K_m$ and $k_{cat}$ were observed, indicating that the function of Factor Xa is not altered by entrapment in DGS-derived silica gel matrix.

Longevity of the enzyme in the DGS-derived silica was also studied. After a ramp up of activity over about 10 days, the activity of the enzyme remained fixed over months (see Figure 9). By contrast, Factor Xa trapped in TEOS-derived silica loses all activity within a few days (see Figure 9).

**(IV) Methods for Preparing Controlled Morphology Silicas**

By combining the new polyol silane precursors of the present invention with appropriate additives and controlled reaction conditions, it is possible to prepare open-cell-structured silica which may be useful for chromatographic assays. The overall pore size, total porosity and surface area of the gels could be changed by adding a variety of different additives. Two different additives were used including:

i) the addition of $Mg^{2+}$ or other multivalent ions, and ii) the addition of hydrophilic polymers of which poly(ethylene oxide) (PEO) is exemplary. It will be appreciated that one or more of these additives may be used in a variety of combinations to control the morphology of the resulting silica.

Accordingly, the present invention relates to a method for preparing a silica monolith comprising:

(a) hydrolyzing and condensing a polyol silane precursor prepared according to the method of the present invention at a pH suitable for the preparation of a silica monolith and in the presence of one or more additives; and
(b) allowing a gel to form.

In embodiments of the present invention, the one or more additives are independently selected from the group consisting of multivalent ions and hydrophilic polymers.

In further embodiments of the present invention, the additive is a multivalent ion. Examples of multivalent ions suitable for use in the method of the invention include, but are not limited to, $Mg^{2+}$. When multivalent metals were added to TEOS and then hydrolyzed, the resulting silica has smaller pores (Example 15).[32] By contrast, in one experiment the preparation of silica from DGS gave average pore sizes of 3.1 nm: the identical recipe (0.027 mol DGS) with the addition of only 0.06 mmol $MgCl_2$ (2.2 mol%) led to significantly larger pores (4.6 nm vs 3.2 nm diameter, Table 7, Figure 10).

In still further embodiments of the present invention the additive is a hydrophilic polymer. Examples of hydrophilic polymers suitable for use in the method of the invention include, but are not limited to, polyols, polysaccharides and poly (ethylene oxide) (PEO). PEO is particularly useful. There was a relationship between the molecular weight and concentration of the PEO used as an additive, and the size and frequencies of pores that were formed in the resulting silica. A comparison of the structures of silica formed from DGS, DGS + 200 MW PEO and DGS + 10000MW PEO is shown in Table 7. Using recipes containing a fixed weight of DGS and PEO, the size of pores increased with PEO molecular weight. Some of the PEO could be removed by washing with water and all the PEO could be removed by pyrolysis

By contrast, additives such as proteins did not behave as porogens When human serum albumin was added to the DGS starting material and hydrolyzed, essentially the same pore sizes and total pore volume was observed as when the protein was not present (Table 7). However, it was also clear that the protein remained trapped inside pores in the monolith: no fluorescently (FITC) labeled human serum albumin could be detected to leach from the column under passive (soaking in a water solution) or active (pumping water through the monolith) conditions. Thus, the proteins were entrapped inside pores and may have formally acted as an additive affecting the pore size (i.e. a porogen). Fluorescent techniques described elsewhere have demonstrated that the entrapped protein is able to move freely: that is, it is not attached physically or chemically to the silica support surface,[33] unlike the case with TEOS-derived glasses.34

**(V) Uses**

The present invention includes the use of a silica monolith prepared using a method of the invention and comprising an active biomolecule entrapped therein, as biosensors, immobilized enzymes or as affinity chromatography supports. Therefore, the present invention relates to the use of a silica monolith comprising an active biomolecule entrapped therein to quantitatively or qualitatively detect a test substance that reacts with or whose reaction is catalyzed by said encapsulated active biomolecule, and wherein said silica monolith is prepared using a method of the invention.

As stated above, the term "biomolecule" includes proteins, peptides, DNA, RNA, whole cells and other such biological substances.

Also included is a method for the quantitative or qualitative detection of a test substance that reacts with or whose reaction is catalyzed by an active biomolecule, wherein said active biomolecule is encapsulated within a silica monolith, and wherein said silica monolith is prepared using a method of the invention. The quantitative/qualitative method comprises (a) preparing a silica monolith comprising said active biological substance entrapped within a silica matrix prepared using a method of the invention; (b) bringing said biomolecule-comprising silica monolith into contact with a gas or aqueous solution comprising the test substance; and (c) quantitatively or qualitatively detecting, observing or measuring the change in one or more optical characteristics in the biomolecule entrapped within the silica monolith.

In particular, the invention includes a method, wherein the change in one or more optical characteristics of the entrapped biomolecule is qualitatively or quantitatively measured by spectroscopy, utilizing one or more techniques selected from the group consisting of UV, IR, visible light, fluorescence, luminescence, absorption, emission. excitation and reflection.

Also included is a method of storing a biologically active biomolecule in a silica matrix, wherein the silica matrix is prepared using a method of the present invention.

The silica monoliths prepared using the method of the invention may also be used in chromatographic applications. For the preparation of a chromatographic column, the silica precursor and, optionally one or more additives and/or a biomolecule, may be placed into a chromatographic column before gelation occurs.

The present invention therefore relates to a method of preparing a chromatographic column comprising:

(c) placing a polyol silane precursor prepared using a method of the invention, in a column, optionally in the presence of one or more additives and/or a biomolecule; and
(d) hydrolyzing and condensing the polyol silane precursor in the column.

In embodiments of the invention, the additives are selected from multivalent ions, such as $Mg^{2+}$ or hydrophilic polymers, such as PEO.

In further embodiments of the invention the chromatographic column is a capillary column. Conventional capillary columns comprise a cylindrical article having an inner wall and an outer wall and involve a stationary phase permanently positioned within a circular cross-section tube having inner diameters ranging from 5 μm to 0.5 mm. The tube wall may be made of glass, metal, plastic and other materials. When the tube wall is made of glass, the wall of the capillary possesses terminal Si-OH groups which can undergo a condensation reaction with terminal Si-OH groups on the silica monolith to produce a covalent "Si-O-Si" linakage between the monolith and the capillary wall. This provides a column with structural integrity that maintains the monolith within the column. Due to the small dimensions of a capillary column, the solutions comprising the silica precursor, and optional additives, may be introduced into the capillary by the application of a modest vacuum.

Some of the additives may be removed or eluted prior to chromatography by rinsing with an appropriate solvent, such as water and/or alcohol. The column may be further prepared by methods such as supercritical drying or the use of a reagent such as a silane or other coupling agent to modify the surface of the exposed silica. The monolith may also

be stored with the additive interspersed within.

In embodiments of the invention, the silica monolith prepared using the method of the invention is further derivatized to allow tailoring of the monolith for a variety of chromatographic separations. For example, a surface may be incorporated into the monolith that is useful for reverse phase chromatography. Such surfaces may comprise long chain alkyl groups or other non-polar groups. Such derivatization may be done by reacting the Si-OH or Si-OR groups on the silica with reagents that convert these functionalities to Si-O linkages to other organic groups such as alkyls. In still further embodiments, the other organic groups are chiral molecules that facilitate the separation of chiral compounds. These derivatizations are known in the art and are included within the scope of the present invention.

The present invention also includes chromatographic columns comprising the silica monoliths prepared as described herein. Accordingly the invention includes a chromatographic column comprising a silica monolith prepared by hydrolyzing and condensing a polyol silane silica precursor, optionally with an additive and/or biological substance, under conditions sufficient for gelation.

The following non-limiting examples are illustrative of the present invention:

## EXAMPLES

### Example 1 - Preparation of glycerylsilane silica precursors

(a) **Diglycerylsilane, DGS (Table 1)**

In a 10 mL round-bottom flask was mixed neat, freshly distilled TEOS (2.08 g, 10.0 mmol) or TMOS (1.52 g, 10.0 mmol)) and glycerol (dried over and distilled from Mg, 1.84 g, 20.0 mmol). The mixture was heated with an oil bath at 130 °C for 36h (TEOS) or at 110 °C for 15h (TMOS) with a reflux condenser in place. Following this time, a stillhead was placed on a short path distillation column and the EtOH or MeOH produced, respectively, was distilled off. Complete removal of EtOH or MeOH and unreacted starting materials at 140 °C *in vacuo* gave DGS that was not contaminated with ethanol or methanol; similar results were observed with other glycerol:silicon ratios. The resulting DGS cannot be purified by normal chromatographic means - hydrolysis competes to form polyglycerylsilanes. The DGS was obtained after all unreacted alcohols were removed by distillation.

**(b) Scale up of (a) to 100g**

The neat mixture of TMOS (76.1 g, 0.5 mol) and glycerol (92.1 g, 1.0 mol) was heated at 105 °C for 5h until the reaction mixture became homogeneous, then the temperature was increased to 110 °C for 47h during which time MeOH was removed by distillation. After distillation stopped, the reaction temperature was increased to 120 °C for 3h: most of mixture turned to hard white solid. Complete removal of MeOH and unreacted starting materials at 140 °C for 2h *in vacuo* gave DGS that was not contaminated with methanol or the analogous alkoxysilanes (as monitored by $^1H$ NMR ($D_2O$)).

The relative amount of $Q^0$ ($Si(OR)_4$) produced, compared to disiloxanes ($Q^1$) and more highly branched siloxanes, as determined by $^{29}Si$ NMR, can be controlled by the amount of contaminant water in the starting TEOS/TMOS and glycerol. In the above experimental protocol, it was crucial to dry the glycerol from Mg, and to freshly distill all other reagents. With very careful drying neither ethoxide or methoxide could be detected by $^1H$ NMR ($D_2O$) in the product.
Yield, 96%, IR 3365m, 2941m, 2887m, 1650w, 1461m, 1417m, 1191s, 1110s, 1051s, 994m, 926m, 859w $cm^{-1}$; $^{13}C$ NMR (MAS) $\delta$ 72.7(m), 63.6(m), 51.9(m) ppm; $^{29}Si$
NMR (MAS) $\delta$ -82.4 (m) ($Q_0$ 97%)[2] -95.6(m) ($Q_2$ 1%) -103.7(m) ($Q_3$ 1%) ppm, appearance, residual OEt (by $^1H$ NMR in $D_2O$), 0%.

**(c) Monoglycerylsilane, MGS (See Table 1)**

The preceding procedure was followed: glycerol (1.84 g, 20.0 mmol); TEOS (3.12 g, 15.0 mmol); Si:glycerol 3:4, Reaction temp., 130 °C; reac. Time, 36h, Yield, 70%, IR 3497s,br, 2924s, 2851s, 2644w, 2179w, 1930m,1739w, 1468m,1403m, 1343w, 1277m, 1222m,1044s, 798w $cm^{-1}$; appearance, wax; residual OEt (by $^1H$ NMR in $D_2O$), 15%.

**(d) Tetraglycerylsilane, TGS (See Table 1)**

The preceding procedure was followed: glycerol (7.37 g, 80.0 mmol); TEOS (4.17 g, 20.0 mmol); Si:glycerol 1:4, Reaction temp., 130 °C; reac. Time, 36h, Yield, 72%, IR 3386s,br, 2941m, 2888m, 1458m,1418m, 1334w, 1262w, 1110s, 1048s, 994m, 926w,857w $cm^{-1}$; appearance, wax; residual OEt (by $^1H$ NMR in $D_2O$), 0%.

**Example 2 - Preparation of sorbitylsilane silica precursors**

**(a) Monosorbitylsilane, MSS (Table 2)**

A DMSO (20 mL) solution of TMOS (1.52 g, 10.0 mmol) and sorbitol (1.82 g, 10.0 mmol) was heated at 120 °C for 48 h, during which, formed MeOH was distilled off. The reaction mixture was concentrated, then added to a large volume of $CH_2Cl_2$. The formed white precipitate was filtered off, washed with $CH_2Cl_2$, and dried at 110 °C *in vacuo* giving sorbityl silanes. If the final step was not utilized, 0-5% MeOSi remained in the **MSS** product. Similar results were observed at other sorbitol:silicon ratios.

**(b) Alternative procedure to MSS avoiding DMSO**

A neat mixture of TMOS (3.04 g, 20.0 mmol) and sorbitol (3.64 g, 20.0 mmol) was heated at 105 °C for 5h until the mixture became homogeneous, then the temperature was increased to 120 °C for 30h, during which time MeOH was distilled off. Completely removal of MeOH and volatile organics at 110°C *in vacuo* gave MSS 3.70 g, (90% yield) that was not contaminated with MeOSi by $^1H$ NMR.
$^{13}C$ CPMAS NMR (300 MHz) $\delta$ 50.9 (br, s), 66.2 (br, m), 72.4 (br, m) ppm; $^{29}Si$ CPMAS NMR (solid state) $\delta$ -80.9 ppm; IR 3432s, 2928m, 1465m, 1441m, 1413m, 1261m, 1068s, 958m, 812m cm$^{-1}$; appearance, white solid; residual OEt (by $^1H$ NMR in $D_2O$), 2.9% (2.9% methoxide remained (by $^1H$ NMR) if a strict 1:1 ratio of sorbitol:TMOS was used. Methoxy groups were completely replaced if a small excess of sorbitol is used).

**(c) Sorbitylsilane2:3, MSS23 (Table 2)**

Either of the preceding procedures was followed: sorbitol (0.36 g, 2.0 mmol); TEOS (0.46 g, 3.0 mmol); Si: sorbitol 3:2, Reaction temp., 120 °C; reac. Time, 48h, Yield, 80% (90% neat), IR 3398s, 2938m, 1458m, 1419w, 1083s, 955m, 818m cm$^{-1}$; appearance, white solid; residual OEt (by $^1H$ NMR in $D_2O$), 1%.

**(d) Disorbitylsilane, DSS (Table 2)**

The preceding DMSO procedure was followed: sorbitol (3.64 g, 20.0 mmol); TEOS (1.52 g, 10.0 mmol); Si: sorbitol 1:2, Reaction temp., 120 °C; reac. Time, 48h, Yield, 77%; IR 3430s, 2939m, 2896m, 1465m, 1447m, 1422m, 1065s, 955m, 891w, 813m cm$^{-1}$; appearance, white solid; residual OEt (by $^1H$ NMR in $D_2O$), 0%.

**Example 3 - Preparation of maltosylsilane silica precursors**

**(a) Maltosyldisilane Ma1S2 (Table 3)**

A DMSO (15 mL) solution of TMOS (0.60 g, 4.0 mmol) and anhydrous maltose anhydride (0.72 g, 2.0 mmol) was heated at 110 °C for 48h, during which time MeOH was distilled off. The reaction mixture was concentrated, then added to large amount of $CH_2Cl_2$, formed white precipitate was filtered off, washed sufficiently with $CH_2Cl_2$, dried at 110 °C *in vacuo* giving Ma1S2. Similar results were observed with different maltoseailicon ratios.

**(b) Maltosyldisilane, Ma1S2 without solvent**

Maltose monohydrate (0.72 g, 2.0 mmol); TEOS (0.60 g, 4.0 mmol); Si: maltose 2:1, Reaction temp., 110 °C; reac. Time, 48h, Yield, 68%; $^{13}C$ CPMAS NMR (solid state) $\delta$ 51.3, 62.2, 73.2, 92.6, 96.5, 102.7 ppm; $^{29}Si$ CPMAS NMR (solid state) $\delta$ -90.8 ppm; IR 3415s, 2927m, 2851w, 1464m, 1447m, 1412m, 1364m, 1320w, 1152s, 1081s, 1048s, 951w, 895w, 836m cm$^{-1}$; appearance, white solid; residual OMe (by $^1H$ NMR in $D_2O$), 0%.

**(c) Monomaltosylsilane, MMS (Table 3)**

The preceding DMSO procedure was followed: maltose monohydrate (3.60 g, 10.0 mmol); TEOS (1.52 g, 10.0 mmol); Si: maltose 1:1, Reaction temp., 110 °C; reac. Time, 48h, Yield, 70%; IR 3409s, 2927m, 2850w, 1439m, 1412m, 1367m, 1324w, 1150m, 1078s, 1036s, 951m, 897w, 842w cm$^{-1}$; appearance, white solid; residual OMe (by $^1H$ NMR in $D_2O$), 1%.

**(d) Dimaltosylsilane, DMS (Table 3)**

The preceding DMSO procedure was followed: maltose monohydrate (7.20 g, 20.0 mmol); TEOS (1.52 g, 10.0 mmol); Si:maltose 1:2, Reaction temp., 110 °C; reac. Time, 48h, Yield, 78%; IR 3394s, 2927m, 2854w, 1438m, 1417m, 1365m, 1320w, 1149m, 1077s, 1036s, 952w, 898w, 840w $cm^{-1}$; appearance, white solid; residual OMe (by $^1H$ NMR in $D_2O$), 0%.

**Example 4 - Preparation of dextransilane (DS) silica precursors (Table 4)**

A DMSO (50 mL) solution of TMOS (4.0 g, 26.3 mmol) and dextran (MW=43,000, 4.3 g, 0.1 mmol) was heated at 120 °C for 48h, during which time MeOH was distilled off. The reaction mixture was concentrated, then added to large amount of dichloromethane, which formed white precipitate that was filtered off, washed sufficiently with $CH_2Cl_2$, and dried at 110 °C *in vacuo* giving **DS**, 4.7 g (95% yield).
$^{13}C$ CPMAS NMR (300 MHz) $\delta$ 51.7, 72.5, 98.3; $^{29}Si$ CPMAS NMR (300 MHz) $\delta$ - 85.5 (85%), -101.8(10%), -109(5%); IR 3410s, 2925m, 2852w, 1644w, 1438m, 1417m, 1356m, 1154vs, 1021vs, 952m, 841w, 764w, 708w, 546w, 457w $cm^{-1}$; appearance, white solid; residual OEt (by $^1H$ NMR in $D_2O$), 0%.

**Example 5 - Preparation of Silica Monolith from Tetraethoxysilane (TEOS)**

**T-1:** TEOS derived gel: TEOS (0.5 g, 2.4 mmol) and HCl solution (0.5 mL, 0.024 M) were mixed with stirring at room temperature. The mixture was allowed to rest for 40 min and then Tris buffer (0.5 mL, 50 mM, pH = 8.25) was added. The gel time after buffer addition was 6.5 min. This protocol was utilized after extensive experimentation of initial pH and water concentration.

**Example 6 - Preparation of Silica Monolith from Diglycerylsilane (DGS) - D-1**

**D-1:** DGS-derived gel: DGS (0.5 g, 2.4 mmol) and $H_2O$ (0.5 ml, 27.8 mmol); the mixture was allowed to rest for 20 min and then Tris buffer (0.5 mL, 50 mM, pH = 8.25) was added. The gel time was 3 min. Note that the slower cure rate data shown in Figure 2 was prepared using more dilute reaction conditions: DGS (0.25 g) + $H_2O$ (750 $\mu$L) + 50 mM Phosphate Buffer (750 $\mu$L).
A series of other monoliths were created from other sugar silanes using a variety of concentrations and pHs using the same basic experimental protocol as for **D-1.** The results are shown in Table 5.

**Example 7 - Preparation of Silica Monolith from Monosorbitylsilane (MSS)**

**M1:** MSS (1.000 g, 4.85 mmol) was either dissolved in HCl (0.1 M, 2.4 ml) or in 2.4 mL of water at pH 7. After sonicating for 10 min, tris Buffer (2.0 mL, 50 mM, pH=8.30) was added. In each case, the transparent gel formed after 3 min.

**Example 8 - Cure Kinetics for DGS as a Function of pH (Figure 2)**

**DGS** (0.2 g) was dissolved in $H_2O$ (600 $\mu$L) in an ultrasonic bath at 0 °C for 15 min until a homogeneous solution formed. Then, buffer (see below, 600 $\mu$L) solution was added. Two vials or cuvettes of the same mixture were prepared at the same time. One for pH or fluorescence measurements, the other was used as reference to determine the gel time. Gel time was determined by the time at which the solution is unable to flow. Solutions of different pH were prepared from standard 5 mM $Na_2HPO_4$ (pH=4.43) and $NaH_2PO_4$ (pH=9.06) phosphate buffers. Note that the morphology of the silica prepared from a sol solution at pH=12.21 was particulate rather than a gel.

**Example 9 - Rate of Cure of TEOS as a Function of Glycerol Concentration**

TEOS (Aldrich, 4.2 g, 20 mmol) was mixed with water (1.4 mL, 78 mmol) and with HCl (0.1 mL, 0.1 M), and then agitated using ultrasound for one hour at 0 °C to give a homogeneous, clear, partially-hydrolyzed TEOS aqueous solution. The pH value was 2.5. The partially hydrolyzed TEOS was used as silicone source for subsequent sol-gel processes.
Aqueous solutions of ethanol (e.g. 12.0 M, 72 $\mu$L, 0.019 mmol), ethylene glycol (8.0M, 72 $\mu$L, 0.0093 mmol) or glycerol (4.0 M, 72 $\mu$L, 0.0031 mmol), respectively, were placed inside the wells of a multi-welled polystyrene plate (see Table 8). Partially hydrolyzed TEOS (100 $\mu$L) was added into each well of polystyrene plate, which contained the mono-, di- and triol, respectively. All samples inside the wells were exposed to an air atmosphere during the sol-gel process. Transparent monolithic silica gels were ultimately obtained: retardation of the gel point in the sol-gel process

was noted (Table 8, Figure 4A).

### Example 10 - Retardation of DGS Cure by Addition of Glycerol

**DGS** (601 mg, 2.89 mmol) was dissolved into water (2.0 g, 111 mmol) to give a 1.44 M solution, which was used as a silicon source for the subsequent sol-gel processes. An aqueous solution of glycerol (Aldrich, 27.79 g dissolved into 100 ml distilled water, (3.0 M) was prepared first. Appropriate dilution of this stock glycerol solution gave other glycerol solutions (2.5 M, 2.0 M, 1.5 M, 1.0 M, 0.5 M, 0.1 M - see Table 10) directly inside wells of a 96-well polystyrene plate. The DGS aqueous solution (300 μL) was added into the aqueous glycerol solutions (100 μL). Neither buffer nor acid were employed. The retardation in gel times is shown in Table 9, Table 10 and Figure 4B.

### Example 11 - Thermogravimetric Analyses (TGA) of DGS derived Silica Gels

Thermogravimetric analysis (see Figure 6 and Figure 7) was performed using a THERMOWAAGE STA409 analyzer. The analysis was measured under air, with flow rate of 50 cc/min. The heat rate was 5 °C/min from room temperature. Freeze drying of samples was accomplished by vacuum treatment of the sample just below 0 °C at 0.2-1 torr. The general procedure used to obtain the results shown in Figure 6 was: All the gels were aged for 2 days at room temperature in the open air, crushed and then freeze-dried at -2 - 0 °C under a vacuum of 0.5-1 torr for 20 hours. The diameter of the monolith was 10 mm. The white powder was directly used as a sample for TGA analysis. The general procedure used to obtain the results shown in Figure 7 was: the gels were prepared by dissolving DGS (0.5-0.6 g, 2.4-2.9 mmol) in $H_2O$ (0.75 mL, 41.7 mmol) and then, after about 10 min, tris Buffer (1 mL, 50 mM, pH=8.35) was added. The gels were aged for 2 days at room temperature, after which: i) the sample was freeze dried at 0 °C ("freeze dried" line; "washed and freeze dried" line; or "soaked in water" line). Details for the "freeze dried" sample are provided in the previous section. The "washed and freeze dried" sample was obtained by crushing the monolith; washing with deionized water for about 2 hours with stirring using a magnetic stirring bar, after which the water was removed by filtration. The washing and filtering was repeated 3 times, and in total, approximately 200 mL $H_2O$ was used. Then, the sample was freeze dried at 0 °C for 20 hours at 0.5-1 torr ("washed and freeze dried" line), after which the TGA was performed). The "monolith soaked in water" sample was obtained by breaking a monolith into several large pieces, which were then soaked in 150 mL deionized water for about 24 hours, and then a second volume of 150 mL water for a further 24 hours. The samples were then taken out from the water, dried in air for 24 hours and then put into a desiccator (anhydrous $CaSO_4$) for 24 hours, after which the TGA was performed ("monolith soaked in water" line).

### Example 12 - Protein Entrapment in DGS derived Silica Monolith

#### (a) Entrapment of Factor Xa in sol-gel matrix:

DGS (0.2 g) was dissolved in water (600 μL) and optionally, HCl (0.1N, 5 μL) was added. This mixture was sonicated in an ice bath for 10 min. The DGS solution (20 μL) was then mixed with Factor Xa in buffer (20 μL, 0.56 μg/mL) in each well of the microtiterplate. Gelation occurred within 5 min. The microtiterplate was then covered with parafilm and a hole was punched through the parafilm on the top of each well. The plate was then stored in a fridge.

#### (b) Enzymatic reaction in solution and in sol-gel matrix

Enzymatic activity of Factor Xa in solution or entrapped in sol-gel was performed in 96 well microtiterplate. For the solution activity test, the substrate solution (200 μL) was modified with varying concentrations of S-2222 (a chromogenic substrate for FactorXa * MERGEFORMAT [35]).Benzamidine was added in each well and the enzyme solution (2 μL, 5.6 μg/mL) was added. The absorbance change at 405 nm was then monitored over 20 min. For the sol-gel entrapped Factor Xa activity test, the sol-gel disk in the well was washed three times with buffer solution. The substrate solution with varying concentration of inhibitor was then added and the absorbance change was monitored at 405 nm for the next 60 min.

The rate of production of 4-nitroaniline as Factor Xa works on the S-2222 substrate, can be monitored at 405 nm, and is therefore a diagnostic of enzyme activity. The enzymatic activity of Factor Xa both in solution (Figure 8a) and in DGS (Figure 8b) follows Michaelis-Menten kinetics. Table 6 summarizes the kinetic values of Factor Xa both in solution and in DGS. No detectable leaching of Factor Xa from the sol-gel matrix was observed.

#### (c) Effect of Ethanol on Factor Xa activity

Factor Xa was incubated in ethanol diluted solutions of 0, 5, 10, 20, 30, 50 and 70 % for two days. Afterwards,

100 μL of the Factor Xa solution and 100 μL of substrate solution were added in each well and the absorbance was monitored at 405 nm. In order to see if the effect of ethanol on Factor Xa activity was reversible, 100 μL of the buffer solution was added into 100 μL of the ethanol/water solutions containing Factor Xa. The resulting solution was incubated for another two days. Afterwards, 100 μL of the resulting solution and 100 μl of substrate solution were added in each well and the absorbance was monitored at 405nm. None of the samples showed any recovery of the activity that was lost upon exposure to ethanol.

**(d) Leaching of Factor Xa from sol-gel matrix**

Buffer solution (50 μl) was added to each well with DGS-derived silica containing Factor Xa and incubated overnight at 4 °C. Afterwards, the supernatant solution was taken out and added to substrate solution to see if any Factor Xa activity could be observed. No activity could be observed, and therefore no detectable leaching of Factor Xa from the sol-gel matrix was observed.

**Example 13: Change in gelation as a function of additives (dopants/porogens)**

Similar recipes were followed as for **D-1** (Example 6), but with additional dopants (additives) added.

**D-2: DGS** (0.5547 g, 2.67 mmol) and $H_2O$ (0.75 mL, 41.7 mmol) were sonicated at 0 °C for about 10 min until the mixture became a homogenous solution. Then tris Buffer (1 mL, 50 mM, pH=8.35) containing human serum albumin (0.1 mM) was added. The transparent gel formed after 5 min. This experiment was repeated with different HSA concentrations. **D-3** is a new silica derived from **DGS** without HSA, **D-4** with 0.5 mM HSA and **D-5** with 1.0 mM HSA.

**D-3: DGS** (1.0 g, 4.81 mmol) and $H_2O$ (1.5 mL, 83.4 mmol) were sonicated at 0 °C for about 10 min until the mixture became a homogenous solution. Then Tris Buffer (1 mL, 50 mM, pH=8.20) was added. The transparent gel formed after 18 min.

**D-4: DGS** (1.0 g, 4.81 mmol) and $H_2O$ (1.5 mL, 83.4 mmol) were sonicated at 0 °C for about 10 min until the mixture became a homogenous solution. Then Tris Buffer (1.5 mL, 50 mM, pH=8.20) containing human serum albumin (0.5 mM) was added. The transparent gel formed after 12 min.

**D-5: DGS** (1.0 g, 4.81 mmol) and $H_2O$ (1.5 mL, 83.4 mmol) were sonicated at 0 °C for about 10 min until the mixture became a homogenous solution. Then Tris Buffer (1 mL, 50 mM, pH=8.20) containing human serum albumin (1.0 mM) was added. The transparent gel formed after 10 min.

**D-6: DGS** (0.5616 g, 2.70 mmol) and aqueous $MgCl_2$ (0.75 mL, 80 mM, 0.06 mmol) were sonicated at 0 °C for about 10 min until the mixture became a homogenous solution. Then tris Buffer (1 mL, 50 mM, pH=8.35) was added. The transparent gel formed after 2 min.

**D-7: DGS** (0.5616 g, 2.69 mmol) and aqueous $MgCl_2$ (0.75 mL, 80 mM, 0.06 mmol) were sonicated at 0 °C for about 10 min until the mixture became a homogenous solution. Then tris Buffer (1 mL, 50 mM, pH=8.35) containing human serum albumin (0.1 mM) was added. The transparent gel formed after 1 min.

**D-8: DGS** (0.56 g, 2.69 mmol) and PEO (0.025 g, MW=200, 12.5 mmol) was added $H_2O$ (0.75 mL, 41.7 mmol). After sonicating at 0 °C for about 10 min the mixture became a homogenous solution at which time TRIS buffer (1.0 mL, 50 mM, pH=8.35) was added. The almost transparent gel (there was some cloudiness) formed after 3 min.

**D-9: DGS** (0.56 g, 2.69 mmol) and PEO (0.025 g, Mw=10000, 2.5 μmol) was added $H_2O$ (0.75 mL, 41.7 mmol). After sonicating at 0 °C for about 10 min the mixture became a homogenous solution at which time TRIS buffer (1.0 mL, 50 mM, pH=8.35) was added. The almost transparent gel (there was some cloudiness) formed after 3 min.

**Example 14 - Pore size analysis**

All samples, after gelation, were aged for two days, washed 3 times with deionized water, freeze dried overnight, and then heated at 200 °C overnight before BET measurements. Samples of T-1 (Example 5), D-1 (Example 6) and M-1 (Example 7), D2-D9 (Example 13) were measured for surface area, pore volume and pore radius with an Autosorb 1 machine from Quantachrome. The samples were evacuated to 0.1 torr before heating. The vacuum was maintained during the outgassing at 200 °C with a final vacuum in the order of 10 millitorr (or less) at completion of the outgassing. The samples were backfilled with helium for removal from the outgas station and prior to analysis. BET surface area was calculated by the BET (Brunauer, Emmett and Teller) equation; the pore size distribution and pore radius nitrogen adsorption-desorption isotherms was calculated by the BJH (Barrett, Joyner and Halenda) method. All the data were calculated by the software provided with the instruments (see Table 7 and Figures 10-11).

**FULL CITATIONS FOR REFERENCES REFERRED TO IN THE SPECIFICATION**

[1] Brook, M.A. Silicon in Organic, Organometallic, and Polymer Chemistry, Wiley: New York, 2000, p. 3.
[2] Stöber, W. F.; Fink, A.; Bohn, E. J. Colloid Interface Sci. 1968, 26, 62.
[3] Blanco, A.; Chomski, E.; Grabtchak, W.; Ibisate, M.; John, S.; Leonard, S. W.; Lopez, C.; Meseguer, F.; Miguez, H.; Mondia, J. P.; Ozin, G. A.; Toader, O.; van Driel, H. M. Nature 2000, 405, 437.
[4] (a) Liu, Y.; Karkamkar, A.; Pinnavaia, T. J. J. Chem. Soc., Chem. Commun. 2001, 1822-1823; (b) Prouzet, E.; Cot, F.; Nabias, G.; Larbot, A.; Kooyman, P.; Pinnavaia, T. J. Chem. Mater. 1999, 11, 1498.
[5] (a) Kresge, C. T.; Leonowicz, M. E.; Roth, W. J.; Vartuli, J. C.; Beck, J. S. Nature 1992, 359, 710; (b) Beck, J. S.; Vartuli, J. C.; Roth, W. J.; Leonowicz, M. E.; Kresge, C. T.; Schmitt, K. D.; Chu, C. T.-W.; Olson, D. H.; Sheppard, E. W.; McCullen, S. B.; Higgins, J. B.; Schlenker, J. L. J. Am. Chem. Soc. 1992, 114, 10834; (c) Inagaki, S.; Fukushima, Y.; Kuroda, K. J. Chem. Soc., Chem. Common. 1993, 680; (d) Chen, C.-Y.; Li, H.-X.; Davis, M. E. Microporous Mater. 1993 2,17; (e) Huo, Q.; Margolese, D. I.; Ciesla, U.; Demuth, D. G.; Feng, P.; Gier, T. E.; Sieger, P.; Firouzi, A.; Chmelka, B. F.; Schith, F.; Stucky, G. D. Chem. Mater. 1994, 6,1176; (f) Huo, Q.; Margolese, D. I.; Ciesla, U.; Feng, P.; Gier, T. E.; Sieger, P.; Leon, R.; Petroff, P. M.; Schüth, F.; Stucky, G. D. Nature 1994, 368, 317; (g) Antonelli, D. M.; Ying, J. Y. Angew. Chem., Int. Ed. Eng. 1996, 35, 426; (h) Antonelli, D. M.; Ying, J. Y. Chem. Mater. 1996, 8, 874; (i) Attard, G. S.; Coleman, N. R. B.; Elliott, J. M. In Mesoporous Molecular Sieves 1998; Bonneviot, L., Beland, F., Danumah, C., Giasson, S., Kaliaguine, S., Eds.; Elsiever Science: Amsterdam, Lausanne, New York, Oxford, Shannon, Singapore, Tokyo, 1998; Vol. 117, p 89; (j) Tanev, P., T.; Chibwe, M.; Pinnavaia, T. J. Nature 1994, 368, 321; (k) Tanev, P., T.; Pinnavaia, T. J. Science 1995, 267, 865; (l) Bagshaw, S. A.; Prouzet, E.; Pinnavaia, T. J. Science 1995, 269, 1242; Bagshaw, S. A.; Pinnavaia, T. J. Angew. Chem., Int. Ed. Eng.1996, 35, 1102; (m) Prouzet, E.; Pinnavaia, T. J. Angew. Chem., Int. Ed. Eng. 1997, 36, 516; (n) Zhao, D.; Feng, J.; Huo, Q.; Melosh, N.; Fredrickson, G. H.; Chmelka, B. F.; Stucky, G. D. Science 1998, 279, 548; (o) Zhao, D.; Huo, Q.; Feng, J.; Chmelka, B. F.; Stucky, G. D. J. Am. Chem. Soc. 1998, 120, 6024; (p) Yang, P.; Deng, T.; Zhao, D.; Feng, P.; Pine, D.; Chmelka, B. F.; Whitesides, G. M.; Stucky, G. D. Science 1998, 282, 2244.
[6] (a) Braun, S.; Rappoport, S.; Zusman, R.; Avnir, D.; Ottolenghi, M. Mater. Lett. 1990, 10, 1. (b) Avnir, D.; Braun, S. Biochemical Aspects of Sol-Gel Science and Technology; Kluwer: Hingham, MA, 1996. (c) Bakul, D. C.; Dunn, B.; Valentine, J. S.; Zink, J. I. Anal. Chem. 1994, 66, 1120. (d) Reetz, M. T.; Zonta, A.; Simpelkamp, J. Angew. Chem., Int. Ed. Engl. 1995, 34, 301. (e) Greaves, M. D.; Rotello, V. M. J. Am. Chem. Soc. 1997, 119, 10569.
[7] For an overview of this area, see: Gill, I., Chem. Mater. 2001, 13, 3404.
[8] (a) Walcarius, A. Electroanalysis 1998, 10, 1217. (b) Lin, J.; Brown, C. W. Trends Anal. Chem. 1997, 16, 200. (c) Tess, M. E.; Cox, J. A. J. Pharm. Biomed. Anal. 1999, 19, 55. (d) Dunn, B.; Miller, J. M.; Dave, B. C.; Valentine, J. S.; Zink, J. I. Acta Mater. 1998, 46, 737. (e) Wang, J. Anal. Chem. Acta 1999, 399, 21. (f) Lan, E. H.; Dave, B. C.; Fukuto, J. M.; Dunn; B.; Zink, J. I.; Valentine, J. S. J. Mater. Chem. 1999, 9, 45. (g) Böttcher, H. J. Prakt. Chem. 2000, 342, 427. (29) Gill, I.; Ballesteros, A. Trends Biotechnol. 2000, 18, 282. (h) Gill, I.; Ballesteros, A. Trends Biotechnol. 2000, 18, 469.
[9] Brinker, C. J.; Scherer, G. W. Sol-Gel Science, Academic Press: San Diego, 1990.
[10] Iler, R. K. The Chemistry of Silica, Wiley: New York, 1979.
[11] Fyfe, C. A.; Aroca, P. P. Chem. Mater. 1995, 7, 1800. (b) Fyfe, C. A.; Aroca, P. P.; Zhang, Y. Bull. Magn. Reson. 1993, 15, 195.
[12] Husing, N.; Schubert U. Angew. Chem. Int. Ed. 1998, 37, 22-45.
[13] (a) Krimm, H.; Schnell, H. German Patent 1136114 (to Farbenfabriken Bayer), 1962; (b) Goldberg, E. P.; Powers, E. J. J. Polym. Sci. Polym. Phys. Ed. 1964, 2, 835; (c) Goneberg, A.; Verheyden, A. Belgian Patent 510419 (to Union chimique belge Soc.), 1952. (d) Vaughn, H. A. British Patent 989379, (to General Electric Co.), 1965. [17] Noll, W. Chemistry and Technology of Silicones, Academic Press, New York, 1968, pp. 371-373.
[18] Spindler, R.; Shriver, D. F. J. Am. Chem. Soc. 1988, 110, 3036; (b) Spindler, R.; Shriver, D. F. Macromolecules 1988, 21, 648.
[19] (a) Cragg, R. H.; Land, R. D. J. Organomet. Chem. 1984, 267, 1; (b) Corriu, R. J-P.; Larchre, F. C.R Hebd. Seances Acad. Sci., Ser. C 1979, 279, 1077; (c) Frye, C. L. J. Org. Chem. 1969, 34, 2496; (d) Mebrotra, R. C.; Narain, R. P. Ind. J. Chem. 1967, 44, 444; (e) Birkofer, L.; Stuhl, J. Organomet. Chem. 1980, 187, 21; (f) Calas, R.; Nicou, P C.R. Hebd. Seances Acad. Sci. 1959, Z49, 1011.
[20] Gill, I.; Ballesteros, A. J. Am. Chem. Soc. 1998, 120, 8587.
[21] (a) Kinrade, S. D.; Maa, K. J.; Schach, A. S.; Sloan, T. A.; Knight, C. T. G. J. Chem. Soc., Dalton Trans. 1999, 3149. (b) Kinrade, S. D.; Hamilton, R. J.; Schach, A. S.; Knight, C. T. G. J. Chem. Soc., Dalton Trans. 2001, 961.
[23] Brinker, C. J.; Scherer, G. W. Sol-Gel Science, Academic Press: San Diego, 1990.
[24] Brook, M. A. Silicon in Organic, Organometallic, and Polymer Chemistry, Wiley: New York, 2000, pp. 12,13.
[25] Liu, Y.; Bolen, D. Biochemistry 1995, 34, 12884.
[26] van Dam-Mieras, M.C.E.; Muller, A.D.; Hemker, H.C. in Methods of Enzymatic Analysis, third edition, Verlag Chemie, vol. 5, pp352-365.
[27] van Dam-Mieras, M.C.E.; Muller, A.D.; van Dieijen, G.; Hemker, H.C. in Methods of Enzymatic Analysis, third

edition, Verlag Chemie, vol. 5, pp365-395
[28] Badjiç, J.D.; Kostiç, N.M. Chem. Mater. 1999,11, 3671-3679.
[29] Williams, A. K.; Hupp, J. T. J. Am. Chem. Soc. 1998, 120, 4366-4371.
[30] Bhatia, R. B.; Brinker, C.J.; Gupta A.K.; Singh, A.K. Chem. Mater. 2000, 12, 2434-2441.
[31] Yamanaka, S.A.; Nguyen, N.P.; Dunn, B.; Valentine; J.S.; Zink, J.I. J. Sol-Gel Sci. Technol. 1996, 7, 117-121.
[32] Murakata, T.; Sato, S.; Ohgawara, T.; Watanabe, T.; Suzuki, T. J. Mater. Sci. 1992, 27, 1567.
[33] Tleugabulova, D.; Brennan, J. D. unpublished data.
[34] Flora, K.K.; Brennan, J.D. Chem. Mater. 2001, 13, 4170.
[35] van Dam-Mieras, M.C.E;. Muller, A.D.; van Dieijen G.; Hemker, H.C. in Methods of Enzymatic Analysis, third edition, Verlag Chemie, vol. 5, pp365-395.

**Table 1: Examples of glyceryl/silane silica precursors**

| | Monoglycerylsilane **MGS** | Diglycerylsilane **DGS** | | Tetraglycerylsilane **TGS** |
|---|---|---|---|---|
| glycerol | 1.84 g, 20.0 mmol | 1.84 g, 20.0 mmol | | 7.37 g, 80.0 mmol |
| alkoxysilane | TEOS 3.12 g, 15.0 mmol | TEOS 2.08 g, 10.0 mmol | TMOS 1.52 g, 10.0 mmol | TEOS 4.17 g, 20.0 mmol |
| Si:glycerol | 3:4 | 1:2 | 1:2 | 1:4 |
| Reaction temperature | 130 °C | 130 °C | 110 °C | 130 °C |
| Reaction time | 36h | 36h | 15h | 36h |
| Yield | 70% | 96% | | 72% |
| $^{13}C$ CPMAS NMR (solid state) $\delta$ | | 72.7(m), 63.6(m), 51.9(m) ppm | | |
| $^{29}Si$ CPMAS NMR (solid state) $\delta$ | | -82.4 (m) ($Q_0$ 97%)[2] -95.6(m) ($Q_2$ 1%) -103.7(m) ($Q_3$ 1%) ppm | | |
| IR | 3497s,br, 2924s, 2851s, 2644w, 2179w, 1930m, 1739w, 1468m,1403m, 1343w, 1277m, 1222m, 1044s, 798w | 3365m, 2941m, 2887m, 1650w, 1461m, 1417m, 1191s, 1110s, 1051s, 994m, 926m, 859w | | 3386s,br, 2941m, 2888m, 1458m,1418m, 1334w, 1262w, 1110s, 1048s, 994m, 926w, 857w |
| | Viscous wax | Colorless solid | | Colorless wax |
| Residual ethoxide or methoxide[1] | 15% | 0% | 0% | 0% |
| [1] as shown by $^{1}H$ NMR ($D_2O$). | | | | |

**Table 2: Examples of sorbityl/silane silane precursors**

| | Sorbitylsilane2:3 **MSS23** | Monosorbitylsilane **MSS** | Disorbitylsilane **DSS** |
|---|---|---|---|
| sorbitol | 0.36 g, 2.0 mmol | 1.82 g, 10.0 mmol | 3.64 g, 20.0 mmol |
| TMOS | 0.46 g, 3.0 mmol | 1.52 g, 10.0 mmol | 1.52 g, 10.0 mmol |
| Si:Sorbitol | 3:2 (Is this ratio correct?) | 1:1 | 1:2 |
| Reaction temperature | 120 °C | 120 °C | 120 °C |
| Reaction time | 48h | 48h | 48h |
| Yield | 80% | 84% | 77% |

(continued)

| | Sorbitylsilane2:3 **MSS23** | Monosorbitylsilane **MSS** | Disorbitylsilane **DSS** |
|---|---|---|---|
| IR | 3398s, 2938m, 1458m, 1419w, 1083s, 955m, 818m | 3432s, 2928m, 1465m, 1441m, 1413m, 1261m, 1068s, 958m, 812m | 3430s, 2939m, 2896m, 1465m, 1447m, 1422m, 1065s, 955m, 891w, 813m |
| Appearance | white solid | white solid | white solid |
| Residual methoxide[1] | 1 | 2.9%[2] | 0% |
| [1] as shown by $^1H$ NMR ($D_2O$).<br>[2] 2.9% methoxide remains (by $^1H$ NMR) if a strict 1:1 ratio of sorbitol:TMOS is used. Methoxy groups are completely replaced if a very small excess of sorbitol is used. | | | |

**Table 3: Examples of maltosyl/silane silica precursors**

| | Maltosyldisilane **Ma1S2** | Monomaltosylsilane **MMS** | Dimaltosylsilane **DMS** |
|---|---|---|---|
| Maltose monohydrate | 0.72 g, 2.0 mmol | 3.60 g, 10.0 mmol | 7.20 g, 20.0 mmol |
| TMOS | 0.60 g, 4.0 mmol | 1.52 g, 10.0 mmol | 1.52 g, 10.0 mmol |
| Si:Maltose | 2:1 | 1:1 | 1:2 |
| Reaction temperature | 110 °C | 110 °C | 110 °C |
| Reaction time | 48h | 48h | 48h |
| Yield | 68% | 70% | 78% |
| IR | 3415s, 2927m, 2851w, 1464m, 1447m, 1412m, 1364m, 1320w, 1152s, 1081s, 1048s, 951w, 895w, 836m | 3409s, 2927m, 2850w, 1439m, 1412m, 1367m, 1324w, 1150m, 1078s, 1036s, 951m, 897w, 842w | 3394s, 2927m, 2854w, 1438m, 1417m, 1365m, 1320w, 1149m, 1077s, 1036s, 952w, 898w, 840w |
| Appearance | White solid | White solid | White solid |
| Residual ethoxide or methoxide[1] | 1.2% OMe | 1% OMe | 0% |
| [1] as shown by $^1H$ NMR ($D_2O$) | | | |

**Table 4: Examples of Dextransilane silica precursors**

| | Dextransilane* (silane:saccharide=1) |
|---|---|
| Dextran, 43000 MW | 4.3 g (0.1 mmol) |
| TEOS or TMOS | TMOS 4.0 g (26.3 mmol) |
| DMSO | 50 mL |
| Si:glycerol | 1:1 |
| Reaction temperature | 120 °C |
| Reaction time | 48h |
| Yield | 95% |
| $^{13}C$ NMR (300 MHz) δ | 51.7, 72.5, 98.3 |
| $^{29}Si$ NMR ($CDCl_3$, 300 MHz) δ | -85.5 (85%), -101.8(10%), -109(5%) |

(continued)

| | Dextransilane* (silane:saccharide=1) |
|---|---|
| Property | Colorless wax |
| IR | 3410s, 2925m, 2852w, 1644w, 1438m, 1417m, 1356m, 1154vs, 1021vs, 952m, 841w, 764w, 708w, 546w, 457w cm- |
| Retaining ethoxide or methoxide[1] | 0% |

**Table 5: Representative gelation experiments with polyol silanes derived from glycerol, sorbitol, maltose and dextran as a function of pH and ionic strength**

| Precursor | DGS | | | | | | | TGS | |
|---|---|---|---|---|---|---|---|---|---|
| | 0.212 g | 0.212 g | 0.212 g | 0.212 g | 0.212 g | 0.212 g | | 0.396 g | 0.396 g |
| $H_2O$ | 300 µL | 300 µL | 300 µL | 300 µL | 500 µL | 500 µL | | 500 µL | 500 µL |
| HCl (0.1 N) | 0 µL | 5 µL | 0 µL | 5 µL | 5 µL | 0 µL | | 0 µL | 0 µL |
| Tris buffer pH 8.0, 50 mM (final conc.) | 0 µL | 0 µL | 300 µL (50mM) | 300 µL (25mM) | 0 µL | 500 µL (25mM) | | 0 µL | 500 µL (25mM) |
| Gel time | 40 | 150 | 5 | 18 | 45 | 10-15 | | 100 | |
| Aging Time | | | | | | 4d | 45d (180d) | 4d | 45d |
| Shrinkage (%v/v) | | | | | | 7 | 50(65) | 4 | 44 |
| Precursor | MSS | | | | | | | | |
| | 0.21g | 0.21g | 0.21g | 0.21g | | | | | |
| $H_2O$ | 600 µL | 300mL | | | | | | | |
| HCl (0.1 N) | | 5 µL | | | | | | | |
| Tris buffer pH 8.0, 50 mM (final | | 300 µL | 600 µL (25mM) | 600 µL | | | | | |
| Gelation | 510 | 50 | 150 | 60 | | | | | |
| Aging | | | 180d 50 | | | | | | |
| Shrinkage (%v/v) | | | | | | | | | |
| Precursor | Maltosyldisilane | | | Monomaltosylsilane | | | Dimaltosylsilane | | |
| | **Ma1S2** | | | **MMS** | | | **DMS** | | |
| | 0.25 g | 0.25 g | 0.25 g | 0.25 g | 0.25 g | 0.25 g | 0.25 g | 0.25 g | 0.25 g |
| $H_2O$ | 600 µL | 300 µL | | 600 µL | 300 µL | | 600 µL | 300 µL | |
| Tris buffer pH 8.0, 50 mM (final | | 300 µL | 600 µL | | 300 µL | 600 µL | | 300 µL | 600 µL |

(continued)

| Precursor | Maltosyldisilane | | | Monomaltosylsilane | | | Dimaltosylsilane | | |
|---|---|---|---|---|---|---|---|---|---|
| Gelation time (min) | 600 | 50 | 45 | NA | 60 | 50 | 2340 | 100 | 80 |

| Precursor | Dextrylsilane (1.1 Si/saccharide unit) |
|---|---|
| | **DS** |
| | 0.24 g |
| $H_2O$ | 500 µL |
| Tris buffer pH 8.0, 50 mM (final | 500 µL |
| Gelation | Did not gel after 10 months |

**Table 6: Kinetic parameters of Factor Xa in solution and in DGS**

| | Km (mM) | $k_{cat}$ ($s^{-1}$) | $k_{cat}$/Km ($M^{-1}$ $s^{-1}$) |
|---|---|---|---|
| Factor Xa in solution | 0.36 | 37 | $10^5$ |
| Factor Xa in DGS | 0.5 | 27 | $4.5\times10^4$ |

**Table 7: Effect of multivalent metals, proteins and PEO on silica pore size when Derived from TEOS (experiment T-1) and DGS (experiments D-1 - D-9)**

| Experiment Number | | Additives | Surface Area Data Single Point BET Data ($m^3$/g) | Pore Volume Data Total pore volume ($cm^3$/g) | Pore Size Data (nm) Average pore radius |
|---|---|---|---|---|---|
| **T-1** | TEOS | | 830 | 0.565 (< 50.0 nm) | 1.29 |
| **D-1** | **DGS** | | 581 | 0.467 (< 56.2 nm) | 1.56 |
| **D-2** | | HSA | 618 | 0.467 (< 50.9 nm) | 1.47 |
| **D-3*** | **DGS** | | 535 | 0.965 (< 53.7 nm) | 3.477 |
| **D-4*** | | HSA (0.5 mM) | 444 | 0.787 (<53.0nm) | 3.432 |
| **D-5*** | | HSA (1 mM) | 450 | 0.838 (<53.9nm) | 3.584 |
| **D-6** | **DGS +** | $MgCl_2$ | 644 | 0.736 (< 53.9 nm) | 2.27 |
| **D-7** | | $MgCl_2$/HSA | 689 | 0.716 (< 45.6 nm) | 2.03 |
| **D-8** | | PEO MW 200 | 565 | 0.476 (<51.2nm) | 1.65 |
| **D-9** | | PEO MW 10k | 560 | 0.506 (<54.2nm) | 1.76 |

*D-3 - D-5 were heated at 500 °C in an oxygen atmosphere before the BET determination.

**Table 8: Relationship between gel time and added alcohols for TEOS-derived silicas**

| [OH] | [EtOH] | Gel time (h) | [$HOCH_2CH_2OH$] | Gel time (h) | [glycerol] | Gel time (h) |
|---|---|---|---|---|---|---|
| 1.5 M | 1.5 M | 12.5 | 1.0 M | 13 | 0.5 M | 13.5 |

(continued)

| [OH] | [EtOH] | Gel time (h) | [$HOCH_2CH_2OH$] | Gel time (h) | [glycerol] | Gel time (h) |
|---|---|---|---|---|---|---|
| 3.0 M | 3.0 M | 12 | 2.0 M | 17 | 1.0 M | 15 |
| 4.5 M | 4.5 M | 11 | 3.0 M | 17 | 1.5 M | 19 |
| 6.0 M | 6.0 M | 9.5 | 4.0 M | 17 | 2.0 M | 19 |
| 9.0 M | 9.0 M | 5 | 6.0 M | 20.5 | 3.0 M | 21.5 |
| 12.0 M | 12.0 M | 3.5 | 8.0 M | 22.5 | 4.0 M | 22.5 |

**Table 9: Relationship between glycerol concentration and gelation time for DGS (DGS concentration held at 1.8 M)**

| Glycerol concentration (M) | Gelation time (min) |
|---|---|
| 0.000 | 275 |
| 0.025 | 277 |
| 0.125 | 300 |
| 0.375 | 330 |
| 0.500 | 335 |
| 0.625 | 339 |
| 0.75 | 345 |

**Table 10: Relationship between glycerol concentration and gelation time for DGS (fluctuating concentration)**

| Entry | 1 | 2 | 3 | 4 | 5 |
|---|---|---|---|---|---|
| DGS | 0.212g | 0.212g | 0.212 g | 0.212 g | 0.212 g |
| Glycerol | 0 g | 0.046 g | 0.092 g | 0.138 g | 0.184 g |
| $H_2O$ | 300$\mu$L | 300$\mu$L | 300$\mu$L | 300$\mu$L | 300$\mu$L |
| DGS:additional glycerol Mole ratio | 1:0 | 1:0.5 | 1:1 | 1:1.5 | 1:2 |
| Gel time (min) | 40 | 75 | 90 | 100 | 100 |
| DGS and glycerol was dissolved in ice-cold water. The mixture left at room temperature to gel. | | | | | |

**Claims**

**1.** A method of preparing organic polyol silanes comprising:

(a) combining at least one alkoxysilane with one or more organic polyols either neat or in the presence of a polar solvent and heating to elevated temperatures for a sufficient period of time for the reaction of the alkoxysilane(s) with the organic polyol(s) to produce polyol-substituted silanes and alkoxy-derived alcohols without the use of a catalyst, wherein the polyol-substitued silanes contain no residual alkoxy groups; and
(b) removal of the alkoxy-derived alcohols,

wherein the one or more organic polyols is selected from the group consisting of sugar alcohols, sugar acids, saccharides, oligosaccharides, polysaccharides, glycerol, propylene glycol and trimethylene glycol and the at least one alkoxysilane is of the formula $R_4Si$, where R is any alkoxy group that may be the same or different and that can be cleaved from silicon under the conditions for performing the method.

2. The method according to claim 1, wherein the one or more alkoxysilanes are selected from the group consisting of tetramethoxysilane, tetraethoxysilane, tetrapropoxysilane tetrabutoxysilane and mixed alkoxysilanes derived from methanol, ethanol, propanol and/or butanol.

3. The method according to claim 2, wherein the one or more alkoxysilanes are selected from the group consisting of tetramethoxysilane and tetraethoxysilane.

4. The method according to any one of claims 1-3, wherein the one or more organic polyols are biomolecule compatile.

5. The method according to claim 4, wherein the biomolecule is a protein, or fragment thereof.

6. The method according to any one of claims 1-5, where the one or more organic polyols is selected from the group consisting of allose, altrose, glucose, mannose, gulose, idose, galactose, talose, ribose, arabinose, xylose, lyxose, threose, erythrosc, glyceraldehydes, sorbose, fructose, dextrose, levulose, sorbitol, sucrose, maltose, cellobiose and lactose, dextran, amylose, pectin, glycerol, propylene glycol and trimethylene glycol.

7. The method according to claim 6, where the one or more organic polyols is selected from the group consisting of glycerol, sorbitol, maltose and dextran.

8. The method according to any one of claims 1-7, wherein the alkoxysilane(s) and organic polyol(s) are heated to a temperature in the range of about 90 °C to about 150 °C for about 3 hours to about 72 hours.

9. The method according to claim 8, wherein the alkoxysilane(s) and organic polyol(s) are heated to a temperature in the range of about 100 °C to about 140 °C for about 10 hours to about 48 hours.

10. An organic polyol silane prepared using the method of any one of claims 1-9.

11. The organic polyol silane according to claim 10, selected from the group consisting of monoglycerylsilane, tetraglycerylsilane, sorbitylsilane2:3, monosorbitylsilane, disorbitylsilane, maltosyldisilane, monomaltosylsilane, dimaltosylsilane, quadridextransilane, demidextransilane and dextransilane.

12. A method for preparing silica monoliths comprising hydrolyzing and condensing an organic polyol silane according to any one of claims 10-11 at a pH in the range of about 5.5 to about 11 and allowing a gel to form.

13. The method according to claim 12, wherein the organic polyol silane is hydrolyzed and condensed in the presence of one or more additives.

14. The method according to claim 13, wherein the one or more additives are independently selected from the group consisting of multivalent ions and hydrophilic polymers.

15. The method according to claim 14, wherein the multivalent ion is $Mg^{2+}$

16. The method according to claim 14, wherein the hydrophilic polymer is selected from the group consisting of polyols, polysaccharides and poly(ethylene oxide) (PEO).

17. The method according to claim 16, wherein the hydrophilic polymer is PEO.

18. The method according to any one of claims 12-17 wherein the polyol silane is hydrolyzed and condensed in the presence of a biomolecule.

19. The method according to claim 18, wherein the biomolecule is selected from the group consisting of proteins, peptides, DNA, RNA and whole cells.

20. The method according to any one of claims 18 and 19, wherein the biomolecule is included in a buffer used to adjust the pH so that it is suitable for the preparation of a silica monolith.

21. A silica monolith prepared using the method according to any one of claims 12-20, wherein the porosity is controlled by one or more additives.

**22.** The monolith according to claim 21, wherein the additives are selected from the group consisting of multivalent ions and hydrophilic polymers,

**23.** The monolith according to claim 22, wherein the hydrophilic polymer is PEO.

**24.** The monolith according to claim 22, wherein the multivalent ion is $Mg^{2+}$.

**25.** A use of a silica monolith comprising an active biomolecule entrapped therein to quantitatively or qualitatively detect a test substance that reacts with or whose reaction is catalyzed by said encapsulated active biomolecule, and wherein said silica monolith is prepared using a method according to any one of claims 18-20.

**26.** The use according to claim 25, wherein the biomolecule is selected from the group consisting of proteins, peptides, DNA, RNA and whole cells.

**27.** A method for the quantitativen or qualitative detection of a test substance that reacts with or whose reaction is catalyzed by an active biomolecule, wherein said active biomolecule is encapsulated within a silica monolith, comprising:

(a) preparing a silica monolith comprising said active biomolecule entrapped within a silica matrix prepared using a method according to any one or claims 18-20;
(b) bringing said biomolecule-comprising silica monolith into contact with a gas or aqueous solution comprising the test substance; and
(c) quantitatively or qualitatively detecting, observing or measuring the change in one or more optical characteristics in the biomolecule entrapped within the silica monolith.

**28.** The method according to claim 27, wherein the change in one or more optical characteristics of the entrapped biomolecule is qualitatively or quantitatively measured by spectroscopy, utilizing one or more techniques selected from the group consisting of UV, IR, visible light, fluorescence, luminescence, absorption, emission. excitation and reflection.

**29.** The use of a silica monolith according to any one of claims 18-20 for storage of a biomolecule in a silica matrix.

**30.** A method for storage of a biomolecule comprising:

(a) preparing a silica monolith comprising said biomolecule entrapped within a silica matrix prepared using a method according to any one or claims 21-28; and
(b) storing said monolith,

**31.** A method of preparing a chromatographic column comprising:

(a) placing a polyol silane precursor prepared using a method according to any one of claims 1-9, in a column, optionally in the presence of one or more additives and/or a biomolecule; and
(b) hydrolyzing and condensing the polyol silane precursor in the column.

**32.** A chromatographic column comprising a silica monoliths prepared using the method according to claim 31.

**Patentansprüche**

**1.** Verfahren zur Herstellung von organischen Polyolsilanen, wobei das Verfahren aufweist:

(a) Vereinigen mindestens eines Alkoxysilans mit einem oder mehreren organischen Polyolen, entweder unverdünnt oder in Gegenwart eines polaren Lösungsmittels, und Erhitzen auf erhöhte Temperaturen während einer ausreichenden Zeitdauer für die Reaktion des (der) Alkoxysilan(e) mit dem (den) organischen Polyol(en), um polyolsubstituierte Silane und alkoxy-derivierte Alkohole ohne Verwendung eines Katalysators zu erzeugen, wobei die polyolsubstituierten Silane keine Restalkoxygruppen enthalten; und
(b) Entfernen der alkoxy-derivierten Alkohole,

wobei das eine oder die mehreren organischen Polyole aus der Gruppe ausgewählt sind, die aus Zuckeralkoholen, Zuckersäuren, Sacchariden, Oligosacchariden, Polysacchariden, Glycerin, Propylenglycol und Trimethylenglycol besteht, und wobei das mindestens eine Alkoxysilan die Formel $R_4Si$ aufweist, wobei R eine Alkoxygruppe ist, die gleich oder unterschiedlich sein kann und die unter den Bedingungen für die Durchführung des Verfahrens von Silicium abgespalten werden kann.

**2.** Verfahren nach Anspruch 1, wobei das eine oder die mehreren Alkoxysilane aus der Gruppe ausgewählt sind, die aus Tetramethoxysilan, Tetraethoxysilan, Tetrapropoxysilan, Tetrabutoxysilan und gemischten Alkoxysilanen besteht, die von Methanol, Ethanol, Propanol und/oder Butanol abgeleitet sind.

**3.** Verfahren nach Anspruch 2, wobei das eine oder die mehreren Alkoxysilane aus der Gruppe ausgewählt sind, die aus Tetramethoxysilan und Tetraethoxysilan besteht.

**4.** Verfahren nach einem der Ansprüche 1-3, wobei das eine oder die mehreren organischen Polyole biomolekülverträglich sind.

**5.** Verfahren nach Anspruch 4, wobei das Biomolekül ein Protein oder ein Fragment davon ist.

**6.** Verfahren nach einem der Ansprüche 1-5, wobei das eine oder die mehreren organischen Polyole aus der Gruppe ausgewählt sind, die aus Allose, Altrose, Glucose, Mannose, Gulose, Idose, Galactose, Talose, Ribose, Arabinose, Xylose, Lyxose, Threose, Erythrose, Glyceraldehyden, Sorbose, Fructose, Dextrose, Levulose, Sorbitol, Sucrose, Maltose, Cellobiose und Lactose, Dextran, Amylose, Pektin, Glycerin, Propylenglycol und Trimethylenglycol besteht.

**7.** Verfahren nach Anspruch 6, wobei das eine oder die mehreren organischen Polyole aus der Gruppe ausgewählt sind, die aus Glycerin, Sorbitol, Maltose und Dextran besteht.

**8.** Verfahren nach einem der Ansprüche 1-7, wobei das eine oder die mehreren Alkoxysilane und organischen Polyole etwa 3 Stunden bis etwa 72 Stunden lang auf eine Temperatur im Bereich von etwa 90°C bis etwa 150°C erhitzt werden.

**9.** Verfahren nach Anspruch 8, wobei das eine oder die mehreren Alkoxysilane und organischen Polyole etwa 10 Stunden bis etwa 48 Stunden lang auf eine Temperatur im Bereich von etwa 100°C bis etwa 140°C erhitzt werden.

**10.** Organisches Poyolsilan, hergestellt unter Anwendung des Verfahrens nach einem der Ansprüche 1-9.

**11.** Organisches Polyolsilan nach Anspruch 10, ausgewählt aus der Gruppe, die aus Monoglycerylsilan, Tetraglycerylsilan, Sorbitylsilan 2:3, Monsorbitylsilan, Disorbitylsilan, Maltosyldisilan, Monomaltosylsilan, Dimaltosylsilan, Quadridextransilan, Demidextransilan und Dextransilan besteht.

**12.** Verfahren zur Herstellung von Silica-Monolithen, das aufweist: Hydrolyse und Kondensation eines organischen Polyolsilans nach einem der Ansprüche 10-11 bei einem pH-Wert im Bereich von etwa 5,5 bis etwa 11 und gelieren lassen.

**13.** Verfahren nach Anspruch 12, wobei das organische Polyolsilan in Gegenwart eines oder mehrerer Zusatzstoffe hydrolysiert und kondensiert wird.

**14.** Verfahren nach Anspruch 13, wobei der eine oder die mehreren Zusatzstoffe unabhängig voneinander aus der Gruppe ausgewählt sind, die aus mehrwertigen Ionen und hydrophilen Polymeren besteht.

**15.** Verfahren nach Anspruch 14, wobei das mehrwertige Ion $Mg^{2+}$ ist.

**16.** Verfahren nach Anspruch 14, wobei das hydrophile Polymer aus der Gruppe ausgewählt ist, die aus Polyolen, Polysacchariden und Poly(ethylenoxid) (PEO) besteht.

**17.** Verfahren nach Anspruch 16, wobei das hydrophile Polymer PEO ist.

**18.** Verfahren nach einem der Ansprüche 12-17, wobei das Polyolsilan in Gegenwart eines Biomoleküls hydrolysiert und kondensiert wird.

**19.** Verfahren nach Anspruch 18, wobei das Biomolekül aus der Gruppe ausgewählt ist, die aus Proteinen, Peptiden, DNA, RNA und intakten Zellen besteht.

**20.** Verfahren nach einem der Ansprüche 18 und 19, wobei das Biomolekül in einem Puffer enthalten ist, der verwendet wird, um den pH-Wert so einzustellen, daß er für die Herstellung eines Silica-Monoliths geeignet ist.

**21.** Silica-Monolith, hergestellt unter Anwendung des Verfahrens nach einem der Ansprüche 12-20, wobei die Porosität durch einen oder mehrere Zusatzstoffe kontrolliert wird.

**22.** Monolith nach Anspruch 21, wobei die Zusatzstoffe aus der Gruppe ausgewählt sind, die aus mehrwertigen Ionen und hydrophilen Polymeren besteht.

**23.** Monolith nach Anspruch 22, wobei das hydrophile Polymer PEO ist.

**24.** Monolith nach Anspruch 22, wobei das mehrwertige Ion $Mg^{2+}$ ist.

**25.** Verwendung eines Silica-Monoliths mit einem darin eingeschlossenen aktiven Biomolekül zum quantitativen oder qualitativen Nachweis einer Testsubstanz, die mit dem eingeschlossenen aktiven Biomolekül reagiert oder deren Reaktion durch dieses katalysiert wird, wobei der Silica-Monolith unter Anwendung eines Verfahrens nach einem der Ansprüche 18-20 hergestellt wird.

**26.** Verwendung nach Anspruch 25, wobei das Biomolekül aus der Gruppe ausgewählt ist, die aus Proteinen, Peptiden, DNA, RNA und intakten Zellen besteht.

**27.** Verfahren für den quantitativen oder qualitativen Nachweis einer Testsubstanz, die mit einem aktiven Biomolekül reagiert oder deren Reaktion durch dieses katalysiert wird, wobei das aktive Biomolekül in einem Silica-Monolith eingeschlossen ist, wobei das Verfahren aufweist:

(a) Herstellung eines Silica-Monoliths mit dem in einer Silica-Matrix eingeschlossenen Biomolekül, hergestellt unter Anwendung eines Verfahrens nach einem der Ansprüche 18-20;
(b) Inkontaktbringen des das Biomolekül aufweisenden Silica-Monoliths mit einem Gas oder einer wäßrigen Lösung, welche die Testsubstanz aufweist; und
(c) quantitativer oder qualitativer Nachweis, Beobachten oder Messen der Veränderung einer oder mehrerer optischer Eigenschaften in dem innerhalb des Silica-Monoliths eingeschlossenen Biomolekül.

**28.** Verfahren nach Anspruch 27, wobei die Veränderung einer oder mehrerer optischer Eigenschaften des eingeschlossenen Biomoleküls qualitativ oder quantitativ durch Spektroskopie unter Anwendung einer oder mehrerer Techniken gemessen wird, die aus der Gruppe ausgewählt sind, die aus UV, IR, sichtbarem Licht, Fluoreszenz, Lumineszenz, Absorption, Emission, Anregung und Reflexion besteht.

**29.** Verwendung eines Silica-Monoliths nach einem der Ansprüche 18-20 zur Lagerung eines Biomoleküls in einer Silica-Matrix.

**30.** Verfahren zur Lagerung eines Biomoleküls, wobei das Verfahren aufweist:

(a) Herstellung eines Silica-Monoliths mit dem in einer Silica-Matrix eingeschlossenen Biomolekül, hergestellt unter Anwendung eines Verfahrens nach einem der Ansprüche 21-28; und
(b) Lagerung des Monoliths.

**31.** Verfahren zur Herstellung einer Chromatographiesäule, wobei das Verfahren aufweist:

(a) Einbringen eines Polyolsilan-Vorläufers, der unter Anwendung eines Verfahrens nach einem der Ansprüche 1-9 hergestellt wird, in eine Säule, wahlweise in Gegenwart eines oder mehrerer Zusatzstoffe und/oder eines Biomoleküls; und
(b) Hydrolyse und Kondensation des Polyolsilan-Vorläufers in der Säule.

**32.** Chromatographiesäule, die einen mit dem Verfahren nach Anspruch 31 hergestellten Silica-Monolith aufweist.

## Revendications

1. Procédé de préparation de polyol silanes organiques comprenant:

(a) la combinaison d'au moins un alcoxysilane avec un ou plusieurs polyol(s) organique(s) soit pur(s) soit en la présence d'un solvant polaire et le chauffage jusqu'à des températures élevées sur une durée suffisante pour que la réaction du(des) alcoxysilane(s) avec le(s) polyol(s) organique(s) produise des silanes substitués par un polyol et des alcools dérivés d'un alcoxy sans l'utilisation d'un catalyseur, dans lequel les silanes substitués par un polyol ne contiennent aucuns groupes alcoxy résiduels ; et
(b) l'élimination des alcools dérivés d'un alcoxy,

dans lequel le un ou plusieurs polyol(s) organique(s) est(sont) choisi(s) parmi le groupe constitué des alcools de sucre, des acides de sucre, des saccharides, des oligosaccharides, des polysaccharides, du glycérol, du propylène glycol et du triméthylène glycol et le au moins un alcoxysilane est de formule $R_4Si$, où R est n'importe quel groupe alcoxy qui peut être identique ou différent et qui peut être clivé à partir du silicium sous les conditions permettant d'effectuer le procédé.

2. Procédé selon la revendication 1, dans lequel le un ou plusieurs alcoxysilane(s) est(sont) choisi(s) parmi le groupe constitué du tétraméthoxysilane, du tétraéthoxysilane, du tétrapropoxysilane tétrabutoxysilane et des alcoxysilanes mixtes dérivés du méthanol, de l'éthanol, du propanol et/ou du butanol.

3. Procédé selon la revendication 2, dans lequel le un ou plusieurs alcoxysilane(s) est(sont) choisi(s) parmi le groupe constitué du tétraméthoxysilane et du tétraéthoxysilane.

4. Procédé selon l'une quelconque des revendications 1 à 3, dans lequel le un ou plusieurs polyol(s) organique(s) est (sont) compatible(s) avec des molécules biologiques.

5. Procédé selon la revendication 4, dans lequel la molécule biologique est une protéine, ou un fragment de celle-ci.

6. Procédé selon l'une quelconque des revendications 1 à 5, où le un ou plusieurs polyol(s) organique(s) est(sont) choisi(s) parmi le groupe constitué de l'allose, de l'altrose, du glucose, du mannose, du gulose, de l'idose, du galactose, du talose, du ribose, de l'arabinose, du xylose, du lyxose, du thréose, de l'érythrose, des glycéraldéhydes, du sorbose, du fructose, du dextrose, du levulose, du sorbitol, du saccharose, du maltose, du cellobiose et du lactose, du dextrane, de l'amylose, de la pectine, du glycérol, du propylène glycol et du triméthylène glycol.

7. Procédé selon la revendication 6, où le un ou plusieurs polyol(s) organique(s) est(sont) choisi(s) parmi le groupe constitué du glycérol, du sorbitol, du maltose et du dextrane.

8. Procédé selon l'une quelconque des revendications 1 à 7, dans lequel le(s) alcoxysilane(s) et le(s) polyol(s) organique (s) est(sont) chauffé(s) à une température située dans la plage d'environ 90°C jusqu'à environ 150°C durant environ 3 heures jusqu'à environ 72 heures.

9. Procédé selon la revendication 8, dans lequel le(s) alcoxysilane(s) et le(s) polyol(s) organique(s) est(sont) chauffé (s) jusqu'à une température située dans la plage d'environ 100°C jusqu'à environ 140°C durant environ 10 heures jusqu'à environ 48 heures.

10. Polyol silane organique préparé en utilisant le procédé selon l'une quelconque des revendications 1 à 9.

11. Polyol silane organique selon la revendication 10, choisi parmi le groupe constitué du monoglycérylsilane, du tétraglycérylsilane, du sorbitylsilane2:3, du monosorbitylsilane, du disorbitylsilane, du maltosyldisilane, du monomaltosylsilane, du dimaltosylsilane, du quadridextransilane, du demidextransilane et du dextransilane.

12. Procédé de préparation de monolithes de silice comprenant l'hydrolyse et la condensation d'un polyol silane organique selon l'une quelconque des revendications 10 à 11 à un pH situé dans la plage d'environ 5,5 à environ 11 et permettant à un gel de se former.

13. Procédé selon la revendication 12, dans lequel le polyol silane organique est hydrolysé et condensé en la présence d'un ou plusieurs additif(s).

**14.** Procédé selon la revendication 13, dans lequel le un ou plusieurs additif(s) est(sont) indépendamment choisi(s) parmi le groupe constitué des ions multivalents et des polymères hydrophiles.

**15.** Procédé selon la revendication 14, dans lequel l'ion multivalent est Mu

**16.** Procédé selon la revendication 14, dans lequel le polymère hydrophile est choisi parmi le groupe constitué des polyols, des polysaccharides et du poly(oxyde d'éthylène) (PEO).

**17.** Procédé selon la revendication 16, dans lequel le polymère hydrophile est le PEO.

**18.** Procédé selon l'une quelconque des revendications 12 à 17, dans lequel le polyol silane est hydrolysé et condensé en la présence d'une molécule biologique.

**19.** Procédé selon la revendication 18, dans lequel la molécule biologique est choisie parmi le groupe constitué des protéines, des peptides, de l'ADN, de l'ARN et de cellules entières.

**20.** Procédé selon l'une quelconque des revendications 18 et 19, dans lequel la molécule biologique est incluse dans un tampon utilisé pour ajuster le pH afin qu'il soit approprié à la préparation d'un monolithe de silice.

**21.** Monolithe de silice préparé en utilisant le procédé selon l'une quelconque des revendications 12 à 20, dans lequel la porosité est contrôlée par un ou plusieurs additif(s).

**22.** Monolithe selon la revendication 21, dans lequel les additifs sont choisis parmi le groupe constitué des ions multivalents et des polymères hydrophiles.

**23.** Monolithe selon la revendication 22, dans lequel le polymère hydrophile est le PEO.

**24.** Monolithe selon la revendication 22, dans lequel l'ion multivalent est $Mg^{2+}$.

**25.** Utilisation d'un monolithe de silice comprenant une molécule biologique active piégée à l'intérieur afin de détecter quantitativement ou qualitativement une substance test qui réagit avec ou dont la réaction est catalysée par ladite molécule biologique active encapsulée, et dans laquelle ledit monolithe de silice est préparé en utilisant un procédé selon l'une quelconque des revendications 18 à 20.

**26.** Utilisation selon la revendication 25, dans laquelle la molécule biologique est choisie parmi le groupe constitué des protéines, des peptides, de l'ADN, de l'ARN et de cellules entières.

**27.** Procédé pour la détection quantitative ou qualitative d'une substance test qui réagit avec ou dont la réaction est catalysée par une molécule biologique active, dans lequel ladite molécule biologique active est encapsulée à l'intérieur d'un monolithe de silice, comprenant:

(a) la préparation d'un monolithe de silice comprenant ladite molécule biologique active piégée à l'intérieur d'une matrice de silice préparée en utilisant un procédé selon l'une quelconque des revendications 18 à 20;
(b) la mise en contact dudit monolithe de silice comprenant la molécule biologique avec une solution gazeuse ou aqueuse comprenant la substance test; et
(c) la détection, observation ou la mesure quantitative ou qualitative du changement au niveau d'une ou plusieurs caractéristique(s) optique(s) dans la molécule biologique piégée à l'intérieur du monolithe de silice.

**28.** Procédé selon la revendication 27, dans lequel le changement au niveau d'une ou plusieurs caractéristique(s) optique(s) de la molécule biologique piégée est mesuré qualitativement ou quantitativement par spectroscopie, en utilisant une ou plusieurs technique(s) choisie(s) parmi le groupe constitué du rayonnement UV, IR, de la lumière visible, de la fluorescence, de la luminescence, de l'absorption, de l'émission, de l'excitation et de la réflexion.

**29.** Utilisation d'un monolithe de silice selon l'une quelconque des revendications 18 à 20 pour le stockage d'une molécule biologique dans une matrice de silice.

**30.** Procédé de stockage d'une molécule biologique comprenant:

(a) la préparation d'un monolithe de silice comprenant ladite molécule biologique piégée à l'intérieur d'une matrice de silice préparée en utilisant un procédé selon l'une quelconque des revendications 21 à 28; et
(b) le stockage dudit monolithe.

**31.** Procédé de préparation d'une colonne chromatographique comprenant:

(a) la mise en place d'un précurseur de polyol silane préparé en utilisant un procédé selon l'une quelconque des revendications 1 à 9, dans une colonne, éventuellement en la présence d'un ou plusieurs additif(s) et/ou d'une molécule biologique; et
(b) l'hydrolyse et la condensation du précurseur de polyol silane dans la colonne.

**32.** Colonne chromatographique comprenant des monolithes de silice préparés en utilisant le procédé selon la revendication 31.

Figure 1

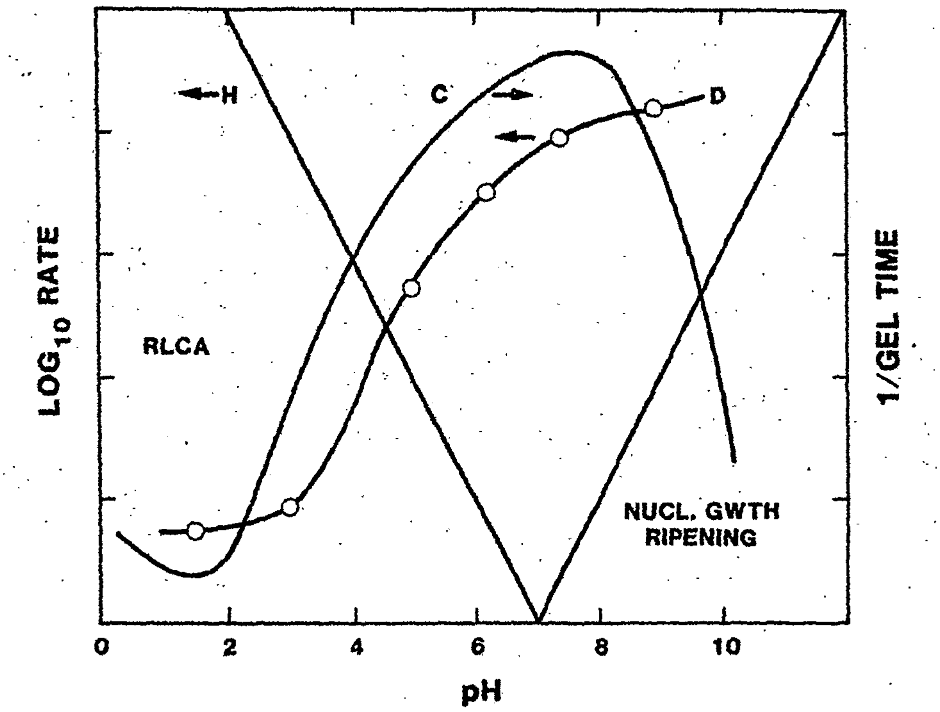

# Figure 2

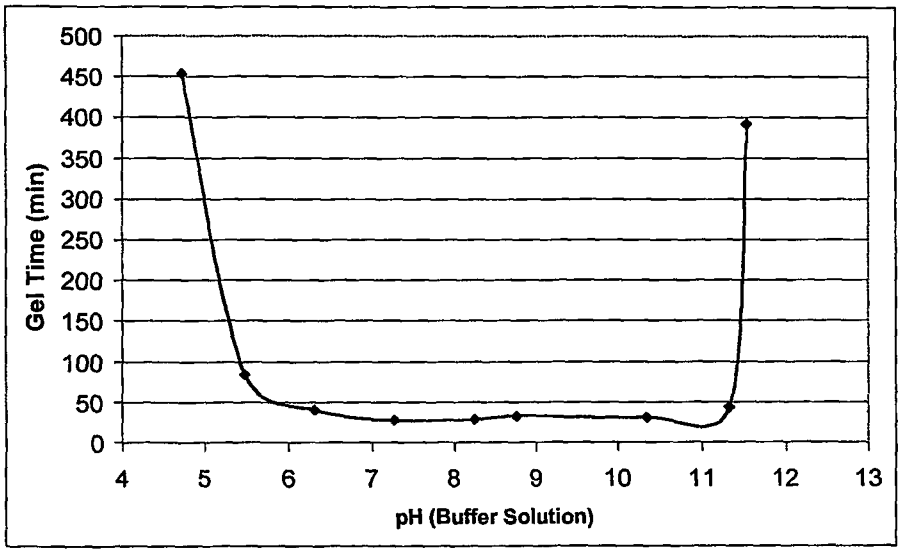
500
450
400
350
300
250
200
150
100
50
0
Gel Time (min)
4
5
6
7
8
9
10
11
12
13
pH (Buffer Solution)

**Figure 3**

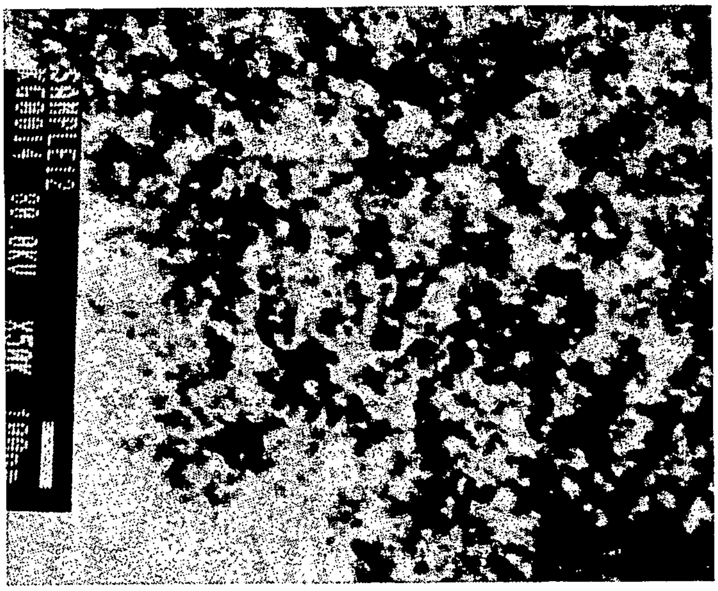

**(vertical scale bar = 100 nm)**

# Figure 4

A:

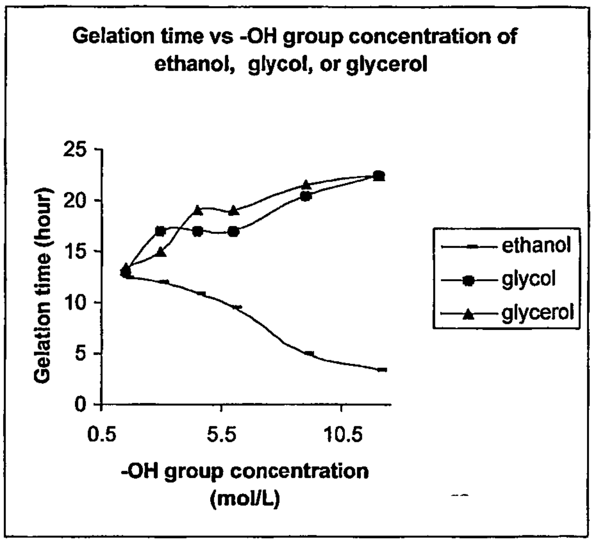
Gelation time vs -OH group concentration of ethanol, glycol, or glycerol
Gelation time (hour)
25
20
15
10
5
0
0.5
5.5
10.5
-OH group concentration (mol/L)
ethanol
glycol
glycerol

B:

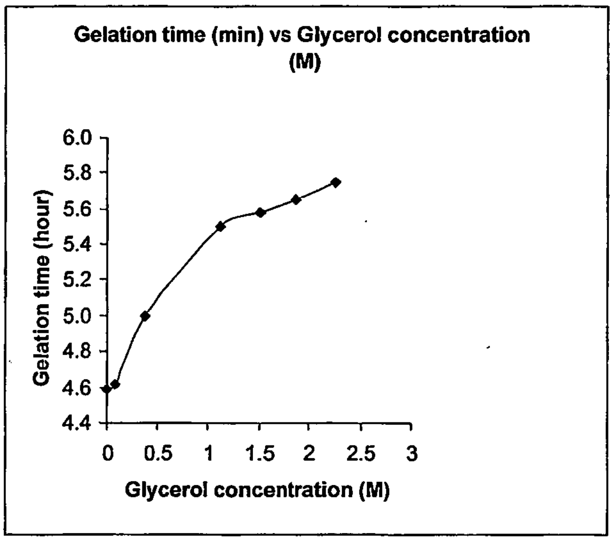
Gelation time (min) vs Glycerol concentration (M)
Gelation time (hour)
6.0
5.8
5.6
5.4
5.2
5.0
4.8
4.6
4.4
0
0.5
1
1.5
2
2.5
3
Glycerol concentration (M)

# Figure 5

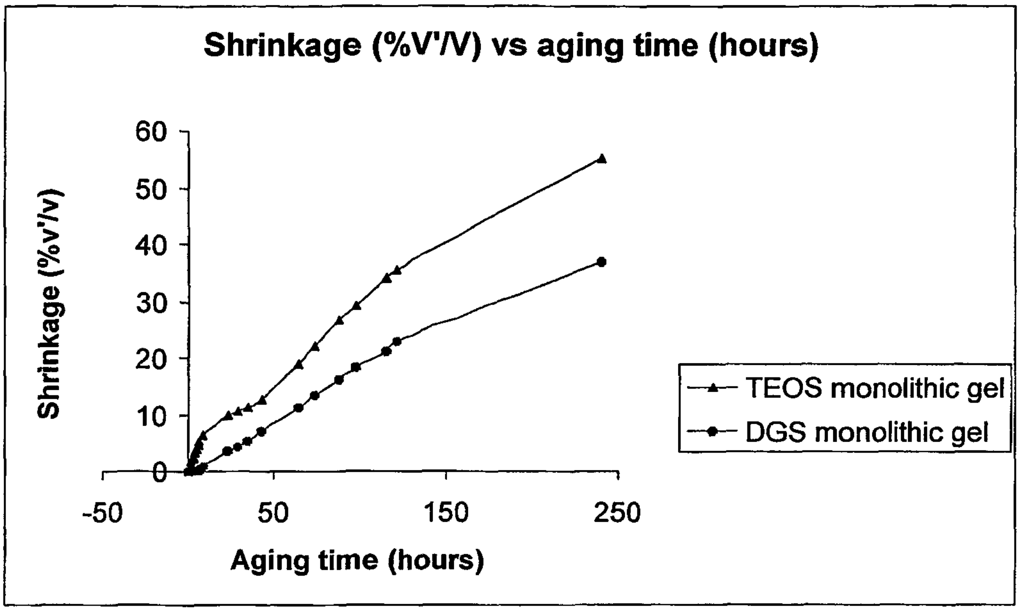
Shrinkage (%V'/V) vs aging time (hours)
Shrinkage (%v'/v)
60
50
40
30
20
10
0
-50
50
150
250
Aging time (hours)
TEOS monolithic gel
DGS monolithic gel

# Figure 6

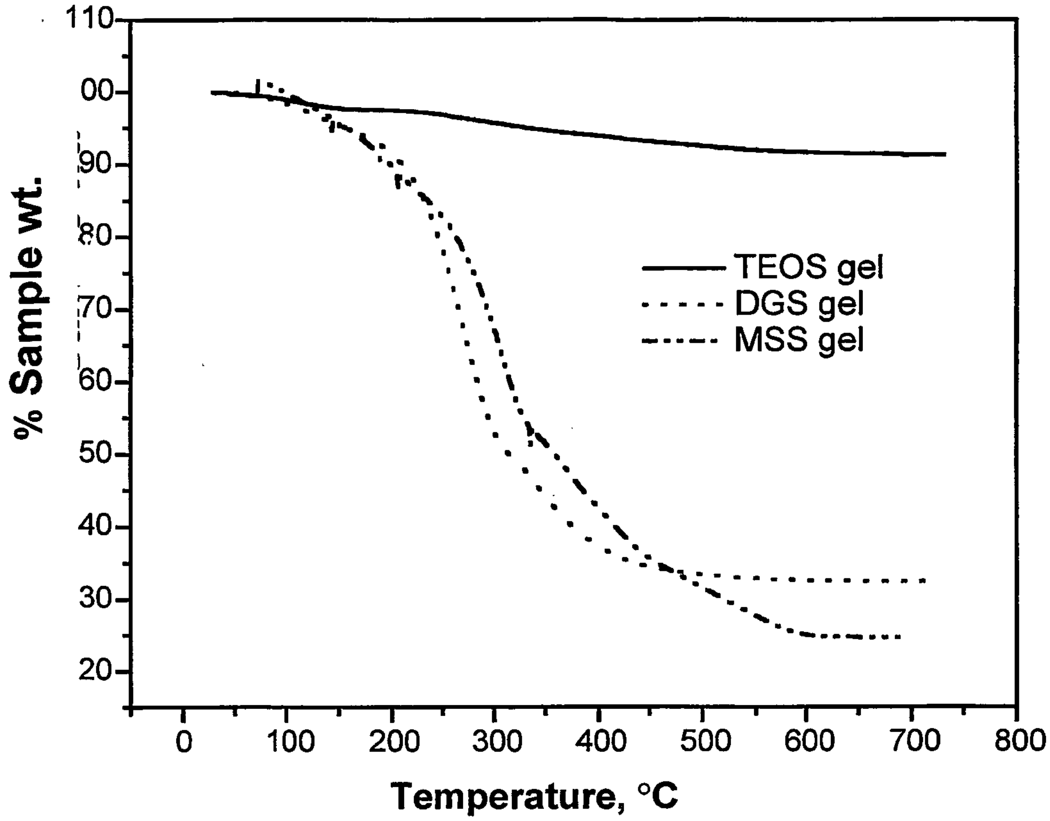
110
00
90
80
70
60
50
40
30
20
% Sample wt.
0
100
200
300
400
500
600
700
800
Temperature, °C
TEOS gel
DGS gel
MSS gel

Figure 7

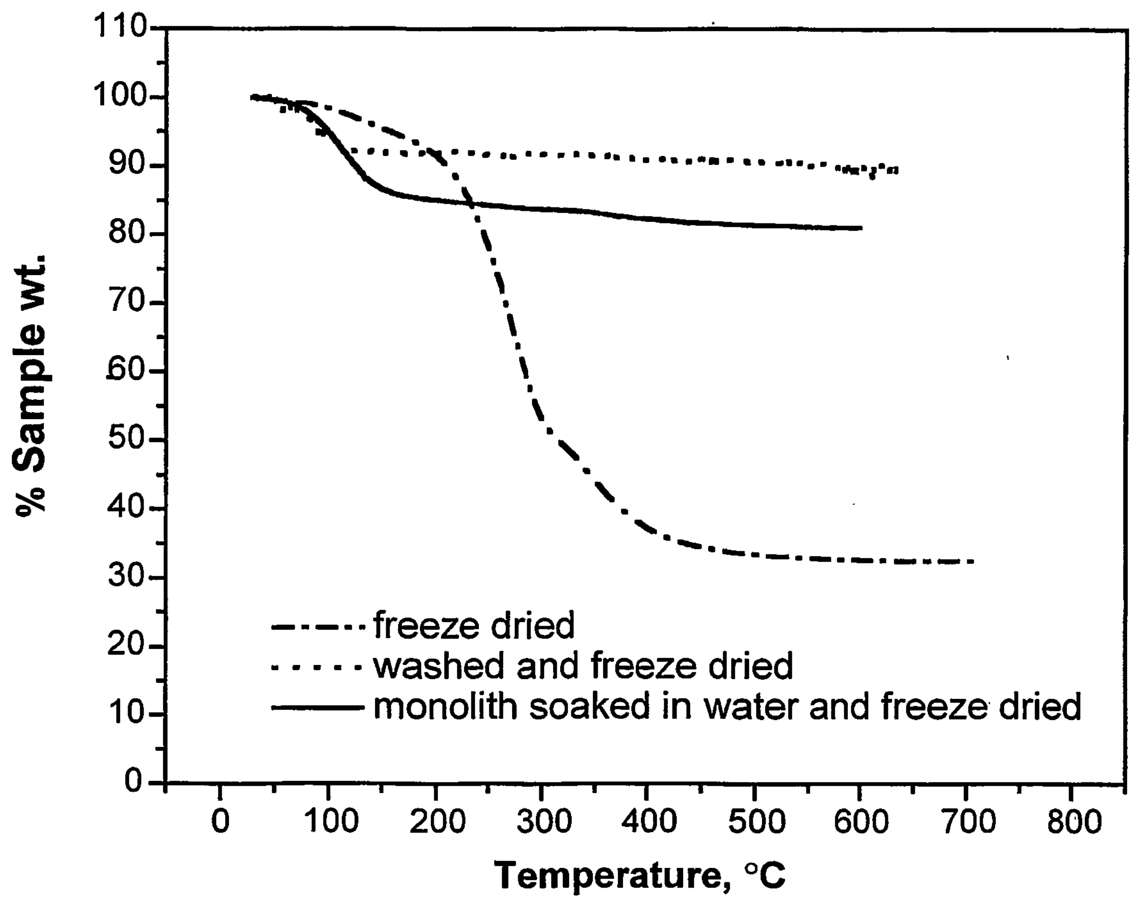
110
100
90
80
70
60
50
40
30
20
10
0
% Sample wt.
0
100
200
300
400
500
600
700
800
Temperature, °C
freeze dried
washed and freeze dried
monolith soaked in water and freeze dried

Figure 8A

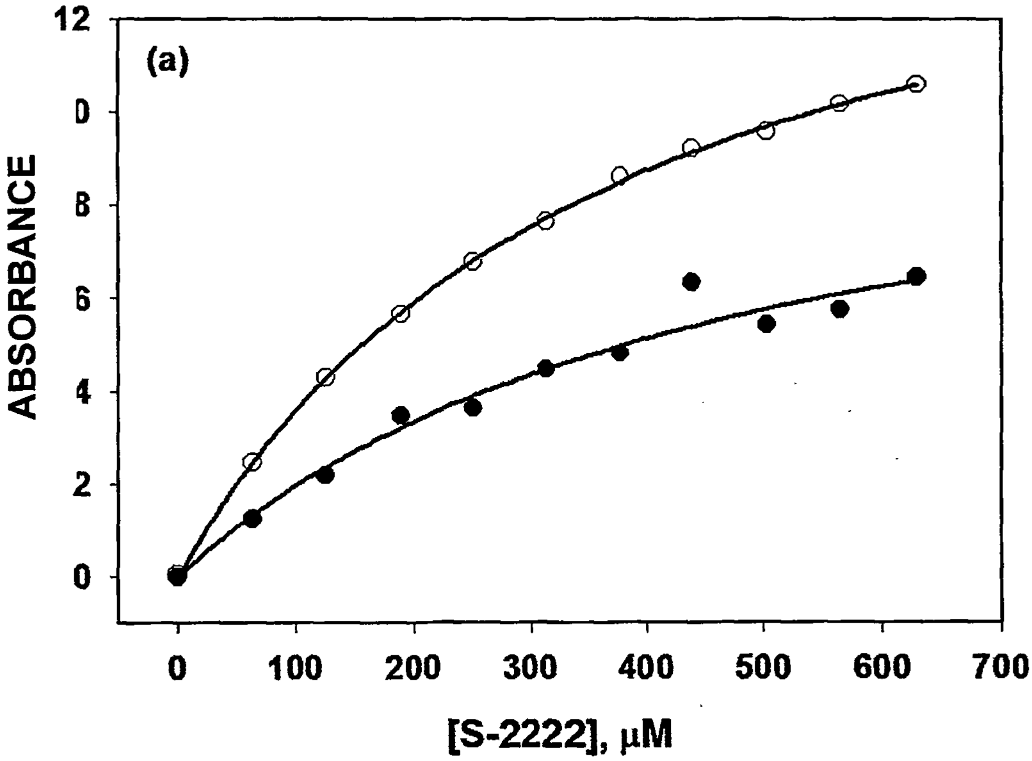

**Figure 8B**

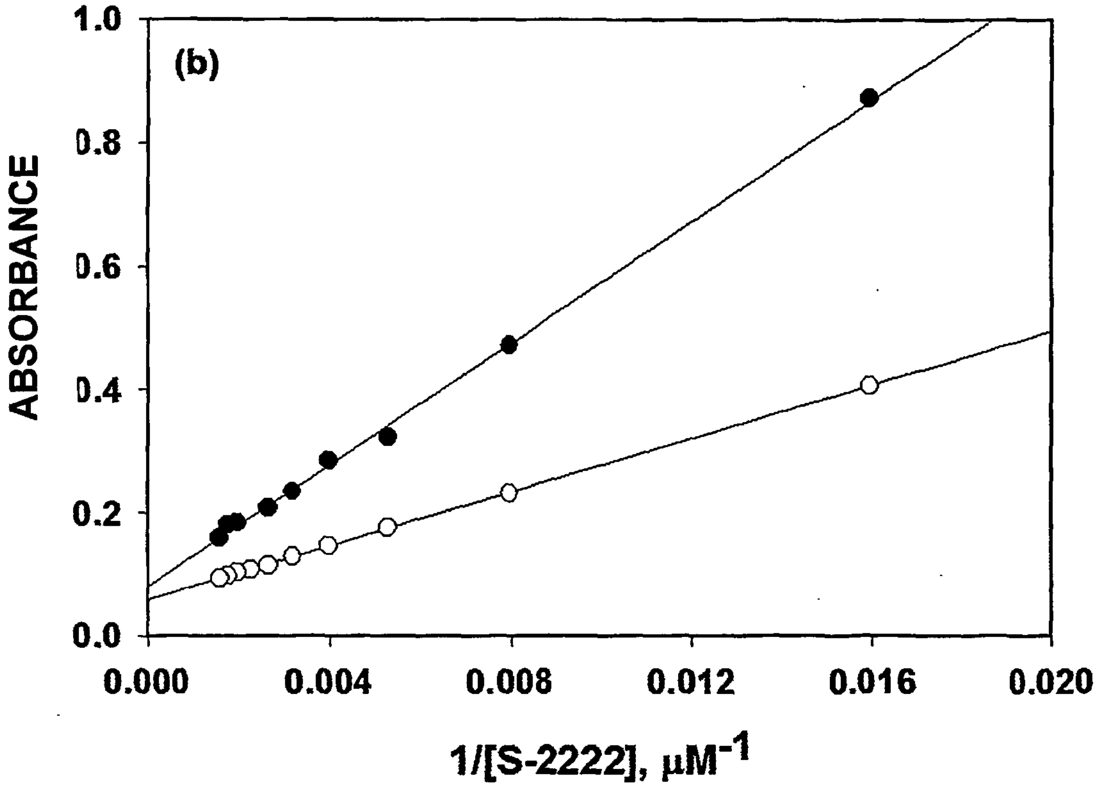
(b)
ABSORBANCE
1.0
0.8
0.6
0.4
0.2
0.0
0.000
0.004
0.008
0.012
0.016
0.020
1/[S-2222], μM-1

**Figure 9**

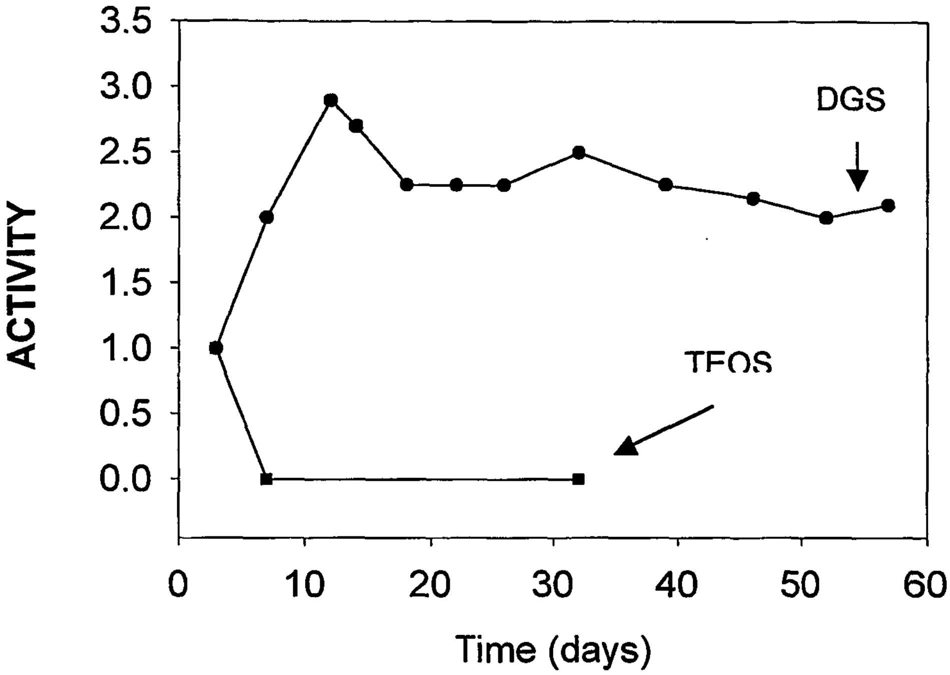
3.5
3.0
2.5
2.0
1.5
1.0
0.5
0.0
ACTIVITY
DGS
TEOS
0
10
20
30
40
50
60
Time (days)

Figure 10

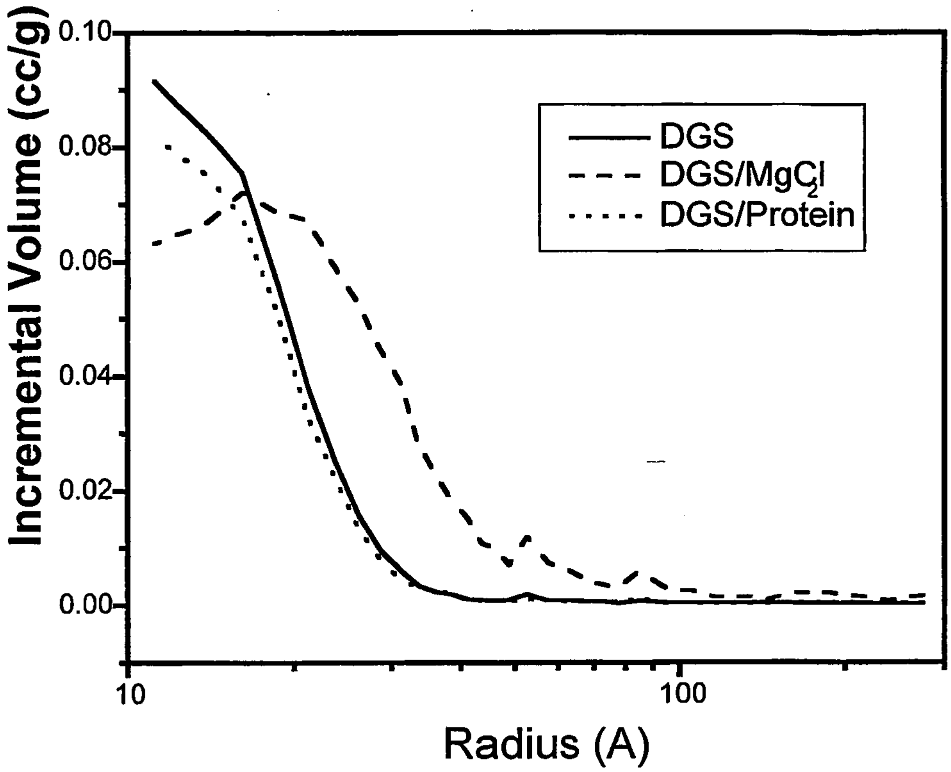
Incremental Volume (cc/g)
0.10
0.08
0.06
0.04
0.02
0.00
10
100
Radius (A)
DGS
DGS/MgCl2
DGS/Protein

**Figure 11**

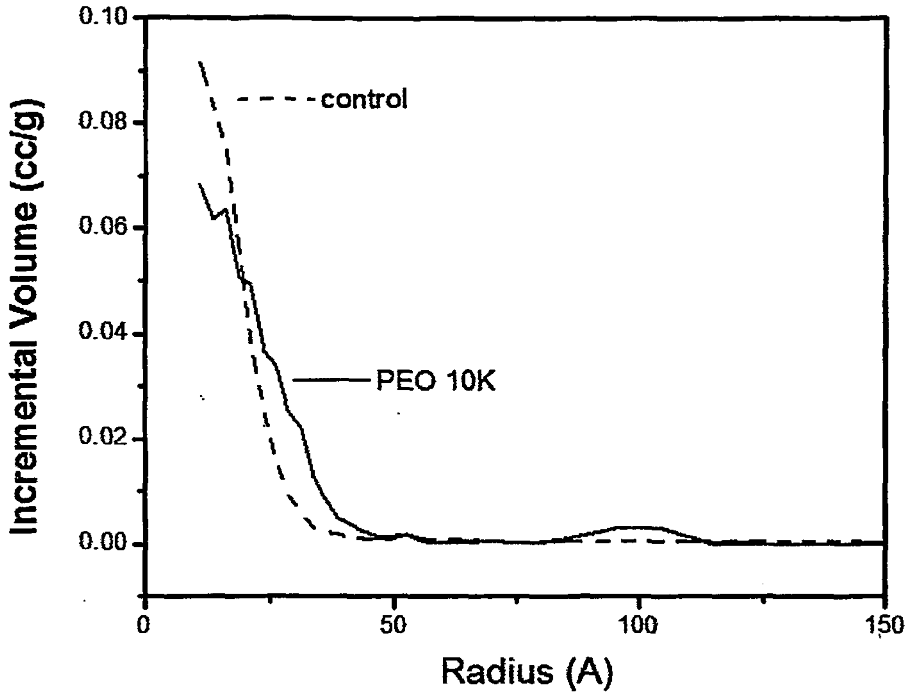
0.10
0.08
0.06
0.04
0.02
0.00
Incremental Volume (cc/g)
control
PEO 10K
0
50
100
150
Radius (A)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9924517 A
- DE 1136114
- BE 510419
- GB 989379 A


### Non-patent literature cited in the description


- **Brook, M.A.** Silicon in Organic, Organometallic, and Polymer Chemistry. Wiley, 2000, 3
- **Stöber, W. F. ; Fink, A. ; Bohn, E.** *J. Colloid Interface Sci.,* 1968, vol. 26, 62
- **Blanco, A. ; Chomski, E. ; Grabtchak, W. ; Ibisate, M. ; John, S. ; Leonard, S. W. ; Lopez, C. ; Meseguer, F. ; Miguez, H. ; Mondia, J. P.** *Nature,* 2000, vol. 405, 437
- **Liu, Y. ; Karkamkar, A. ; Pinnavaia, T. J.** *J. Chem. Soc., Chem. Commun.,* 2001, 1822-1823
- **Prouzet, E. ; Cot, F. ; Nabias, G. ; Larbot, A. ; Kooyman, P. ; Pinnavaia, T.** *J. Chem. Mater.,* 1999, vol. 11, 1498
- **Kresge, C. T. ; Leonowicz, M. E. ; Roth, W. J. ; Vartuli, J. C. ; Beck, J. S.** *Nature,* 1992, vol. 359, 710
- **Beck, J. S. ; Vartuli, J. C. ; Roth, W. J. ; Leonowicz, M. E. ; Kresge, C. T. ; Schmitt, K. D. ; Chu, C. T.-W. ; Olson, D. H. ; Sheppard, E. W. ; McCullen, S. B.** *J. Am. Chem. Soc.,* 1992, vol. 114, 10834
- **Inagaki, S. ; Fukushima, Y. ; Kuroda, K.** *J. Chem. Soc., Chem. Common.,* 1993, 680
- **Chen, C.-Y. ; Li, H.-X. ; Davis, M. E.** *Microporous Mater.,* 1993, vol. 2, 17
- **Huo, Q. ; Margolese, D. I. ; Ciesla, U. ; Demuth, D. G. ; Feng, P. ; Gier, T. E. ; Sieger, P. ; Firouzi, A. ; Chmelka, B. F. ; Schith, F.** *Chem. Mater.,* 1994, vol. 6, 1176
- **Huo, Q. ; Margolese, D. I. ; Ciesla, U. ; Feng, P. ; Gier, T. E. ; Sieger, P. ; Leon, R. ; Petroff, P. M. ; Schüth, F. ; Stucky, G. D.** *Nature,* 1994, vol. 368, 317
- **Antonelli, D. M. ; Ying, J. Y.** *Angew. Chem., Int. Ed. Eng.,* 1996, vol. 35, 426
- **Antonelli, D. M. ; Ying, J. Y.** *Chem. Mater.,* 1996, vol. 8, 874
- **Attard, G. S. ; Coleman, N. R. B. ; Elliott, J. M.** Mesoporous Molecular Sieves. Elsiever Science, 1998, vol. 117, 89
- **Tanev, P., T. ; Chibwe, M. ; Pinnavaia, T. J.** *Nature,* 1994, vol. 368, 321
- **Tanev, P., T. ; Pinnavaia, T. J.** *Science,* 1995, vol. 267, 865
- **Bagshaw, S. A. ; Prouzet, E. ; Pinnavaia, T. J.** *Science,* 1995, vol. 269, 1242
- **Bagshaw, S. A. ; Pinnavaia, T. J.** *Angew. Chem., Int. Ed. Eng.,* 1996, vol. 35, 1102
- **Prouzet, E. ; Pinnavaia, T. J.** *Angew. Chem., Int. Ed. Eng.,* 1997, vol. 36, 516
- **Zhao, D. ; Feng, J. ; Huo, Q. ; Melosh, N. ; Fredrickson, G. H. ; Chmelka, B. F. ; Stucky, G. D.** *Science,* 1998, vol. 279, 548
- **Zhao, D. ; Huo, Q. ; Feng, J. ; Chmelka, B. F. ; Stucky, G. D.** *J. Am. Chem. Soc.,* 1998, vol. 120, 6024
- **Yang, P. ; Deng, T. ; Zhao, D. ; Feng, P. ; Pine, D. ; Chmelka, B. F. ; Whitesides, G. M. ; Stucky, G. D.** *Science,* 1998, vol. 282, 2244
- **Braun, S. ; Rappoport, S. ; Zusman, R. ; Avnir, D. ; Ottolenghi, M.** *Mater. Lett.,* 1990, vol. 10, 1
- **Avnir, D. ; Braun, S.** Biochemical Aspects of Sol-Gel Science and Technology. Kluwer, 1996
- **Bakul, D. C. ; Dunn, B. ; Valentine, J. S. ; Zink, J. I.** *Anal. Chem.,* 1994, vol. 66, 1120
- **Reetz, M. T. ; Zonta, A. ; Simpelkamp, J.** *Angew. Chem., Int. Ed. Engl.,* 1995, vol. 34, 301
- **Greaves, M. D. ; Rotello, V. M.** *J. Am. Chem. Soc.,* 1997, vol. 119, 10569
- **Gill, I.** *Chem. Mater.,* 2001, vol. 13, 3404
- **Walcarius, A.** *Electroanalysis,* 1998, vol. 10, 1217
- **Lin, J. ; Brown, C. W.** *Trends Anal. Chem.,* 1997, vol. 16, 200
- **Tess, M. E. ; Cox, J. A.** *J. Pharm. Biomed. Anal.,* 1999, vol. 19, 55
- **Dunn, B. ; Miller, J. M. ; Dave, B. C. ; Valentine, J. S. ; Zink, J. I.** *Acta Mater.,* 1998, vol. 46, 737
- **Wang, J.** *Anal. Chem. Acta,* 1999, vol. 399, 21
- **Dave, B. C. ; Fukuto, J. M. ; Dunn; B. ; Zink, J. I. ; Valentine, J. S.** *J. Mater. Chem.,* 1999, vol. 9, 45
- **Böttcher, H.** *J. Prakt. Chem.,* 2000, vol. 342, 427

- **Gill, I. ; Ballesteros, A.** *Trends Biotechnol.,* 2000, vol. 18, 282
- **Gill, I. ; Ballesteros, A.** *Trends Biotechnol.,* 2000, vol. 18, 469
- **Brinker, C. J. ; Scherer, G. W.** Sol-Gel Science. Academic Press, 1990
- **Iler, R. K.** The Chemistry of Silica. Wiley, 1979
- **Fyfe, C. A. ; Aroca, P. P.** *Chem. Mater.,* 1995, vol. 7, 1800
- **Fyfe, C. A. ; Aroca, P. P. ; Zhang, Y.** *Bull. Magn. Reson.,* 1993, vol. 15, 195
- **Husing, N. ; Schubert U.** *Angew. Chem. Int. Ed.,* 1998, vol. 37, 22-45
- **Goldberg, E. P. ; Powers, E. J.** *J. Polym. Sci. Polym. Phys. Ed.,* 1964, vol. 2, 835
- **Noll, W.** Chemistry and Technology of Silicones. Academic Press, 1968, 371-373
- **Spindler, R. ; Shriver, D. F.** *J. Am. Chem. Soc.,* 1988, vol. 110, 3036
- **Spindler, R. ; Shriver, D. F.** *Macromolecules,* 1988, vol. 21, 648
- **Cragg, R. H. ; Land, R. D.** *J. Organomet. Chem.,* 1984, vol. 267, 1
- **Corriu, R. J-P. ; Larchre, F.** *C.R Hebd. Seances Acad. Sci., Ser. C,* 1979, vol. 279, 1077
- **Frye, C. L.** *J. Org. Chem.,* 1969, vol. 34, 2496
- **Mebrotra, R. C. ; Narain, R. P.** *Ind. J. Chem.,* 1967, vol. 44, 444
- **Birkofer, L. ; Stuhl, J.** *Organomet. Chem.,* 1980, vol. 187, 21
- **Calas, R. ; Nicou, P.** *C.R. Hebd. Seances Acad. Sci.,* 1959, vol. Z49, 1011
- **Gill, I. ; Ballesteros, A.** *J. Am. Chem. Soc.,* 1998, vol. 120, 8587
- **Kinrade, S. D. ; Maa, K. J. ; Schach, A. S. ; Sloan, T. A. ; Knight, C. T. G.** *J. Chem. Soc., Dalton Trans.,* 1999, 3149
- **Kinrade, S. D. ; Hamilton, R. J. ; Schach, A. S. ; Knight, C. T. G.** *J. Chem. Soc., Dalton Trans.,* 961
- Silicon in Organic, Organometallic, and Polymer Chemistry. **Brook, M. A.** Silicon in Organic, Organometallic, and Polymer Chemistry. Wiley, 2000, 12, 13
- **Liu, Y. ; Bolen, D.** *Biochemistry,* 1995, vol. 34, 12884
- **van Dam-Mieras, M.C.E. ; Muller, A.D. ; Hemker, H.C.** Methods of Enzymatic Analysis. Verlag Chemie, vol. 5, 352-365
- **van Dam-Mieras, M.C.E. ; Muller, A.D. ; van Dieijen, G. ; Hemker, H.C.** Methods of Enzymatic Analysis. Verlag Chemie, vol. 5, 365-395
- **Badjiç, J.D. ; Kostiç, N.M.** *Chem. Mater.,* 1999, vol. 11, 3671-3679
- **Williams, A. K. ; Hupp, J. T.** *J. Am. Chem. Soc.,* 1998, vol. 120, 4366-4371
- **Bhatia, R. B. ; Brinker, C.J. ; Gupta A.K. ; Singh, A.K.** *Chem. Mater.,* 2000, vol. 12, 2434-2441
- **Yamanaka, S.A. ; Nguyen, N.P. ; Dunn, B. ; Valentine; J.S. ; Zink, J.I.** *J. Sol-Gel Sci. Technol.,* 1996, vol. 7, 117-121
- **Murakata, T. ; Sato, S. ; Ohgawara, T. ; Watanabe, T. ; Suzuki, T.** *J. Mater. Sci.,* 1992, vol. 27, 1567
- **Flora, K.K. ; Brennan, J.D.** *Chem. Mater.,* 2001, vol. 13, 4170
- **van Dam-Mieras, M.C.E ; Muller, A.D. ; van Dieijen G. ; Hemker, H.C.** Methods of Enzymatic Analysis. Verlag Chemie, vol. 5, 365-395